# EUROPEAN PATENT APPLICATION

(11) **EP 4 198 129 A1**
(43) Date of publication of application: **21.06.2023**
(21) Application number: 21861695.1
(22) Date of filing: 27.08.2021
(51) Int. Cl.: C12N 15/56, C12N 5/10, C12N 9/24, C12N 15/12, C12N 15/63

(54) **MUTANT OF ALPHA-N-ACETYLGLUCOSAMINIDASE**

(30) Priority: 28.08.2020 JP 2020145100
(71) Applicant: JCR Pharmaceuticals Co., Ltd., Ashiya-shi, Hyogo 659-0021 (JP)
(72) Inventor: TAKAHASHI, Kenichi, Kobe-shi, Hyogo 651-2241 (JP); KUBOTA, Saki, Kobe-shi, Hyogo 651-2241 (JP); Zolotaryov, Fyodor N., Kobe-shi, Hyogo 651-2241 (JP)
(74) Representative: Boult Wade Tennant LLP
(86) International application number: PCT/JP2021/031431
(87) International publication number: WO 2022/045273

(57) **Abstract**

The present invention relates to a mutant of human a-N-acetylglucosaminidase (hNAGLU), more specifically a hNAGLU mutant produced by adding a mutation to an amino acid sequence for hNAGLU such that an expression level of hNAGLU in a host cell in which a gene encoding hNAGLU is introduced can be increased compared with the case where a gene encoding wild-type hNAGLU is introduced. For example, the hNAGLU mutant has an amino acid sequence represented by SEQ ID NO: 3, SEQ ID NO: 5, SEQ ID NO: 9, SEQ ID NO: 11, SEQ ID NO: 15, or SEQ ID NO: 19, or has an amino acid sequence introduced a mutation to the amino acid sequence of any one of the hNAGLU mutants.

## Description

### Technical Field

The present invention relates to a mutant of human α-N-acetylglucosaminidase (hNAGLU), more specifically a new hNAGLU mutant produced by adding a mutation to an amino acid sequence for hNAGLU in order that an expression level of hNAGLU in a host cell in which a gene encoding hNAGLU is introduced can be increased compared with the case where a gene encoding wild-type hNAGLU is introduced.

### Background Art

Mucopolysaccharidosis type IIIB (MPS-IIIB), which is one of lysosomal diseases, is known as Sanfilippo syndrome type B, and is a hereditary disease caused by decreased or deficient enzyme activity, which is associated with genetic abnormality of the enzyme α-N-acetylglucosaminidase (NAGLU), an enzyme required for the decomposition of heparan sulfate (HS), which is a type of glycosaminoglycan (GAG), in lysosomes. In severe cases, HS accumulation in various organs, including the brain, causes severe neurological and tissue disorders such as cognitive decline and behavioral disorders between the ages of 2 and 6, and behavioral disorders such as hyperactivity and mental retardation are observed. Severe mental retardation and motor disorders associated with rapid progression of central neurodegenerative symptoms are observed, and language ability disappears by the age of 7 to 8. During the teenage years, sleep disturbance, hepatosplenomegaly, and convulsive seizures are observed, and due to incapacitation and bedridden condition, most die of respiratory infections in their twenties.

Enzyme replacement therapy has been used to replace insufficient or deficient enzymes in patients with Sanfilippo syndrome type B. The enzyme α-N-acetylglucosaminidase (NAGLU) has the effect of catalyzing the hydrolysis reaction of non-reducing terminal α-N-acetylglucosamine residues of heparan sulfate, and can decompose HS accumulated in lysosomes in the patient's body when administered to a patient.

A gene encoding wild-type human NAGLU (hNAGLU) was isolated in 1995 (Patent Literature 1). hNAGLU used in enzyme replacement therapy is recombinant hNAGLU produced using cells transformed with an expression vector incorporating the gene encoding hNAGLU.

### Citation List

### Patent Literature

[Patent Literature 1] Japanese Unexamined Patent Publication No. 2000-500972

### Summary of Invention

### Technical Problem

An object of the present invention is to provide a new hNAGLU mutant produced by adding a mutation to an amino acid sequence for hNAGLU such that an expression level of hNAGLU in a host cell in which a gene encoding hNAGLU is introduced can be increased compared with the case where a gene encoding wild-type hNAGLU is introduced.

### Solution to Problem

In the research for the above purpose, as a result of extensive studies, the present inventors have found that a host cell in which a gene encoding a hNAGLU mutant obtained by modifying an amino acid sequence of hNAGLU is introduced expresses more hNAGLU than a host cell in which a gene encoding wild-type hNAGLU is introduced, and have completed the present invention. That is, the present invention includes the following.
1. A mutant of human α-N-acetylglucosaminidase (hNAGLU) selected from the group consisting of (1) to (7) below:
   (1) a mutant containing an amino acid sequence represented by SEQ ID NO: 3 in which lysine at position 36 in a wild-type hNAGLU amino acid sequence represented by SEQ ID NO: 1 is replaced with glutamic acid and proline at position 37 is replaced with serine, respectively;
   (2) a mutant containing an amino acid sequence represented by SEQ ID NO: 5 in which serine is added between leucine at position 44 and glycine at position 45 in the wild-type hNAGLU amino acid sequence represented by SEQ ID NO: 1;
   (3) a mutant containing an amino acid sequence represented by SEQ ID NO: 9 in which glutamine at position 209 in the wild-type hNAGLU amino acid sequence represented by SEQ ID NO: 1 is replaced with arginine;
   (4) a mutant containing an amino acid sequence represented by SEQ ID NO: 11 in which glutamic acid at position 228 in the wild-type hNAGLU amino acid sequence represented by SEQ ID NO: 1 is replaced with lysine;
   (5) a mutant containing an amino acid sequence represented by SEQ ID NO: 15 in which threonine at position 320 in the wild-type hNAGLU amino acid sequence represented by SEQ ID NO: 1 is replaced with proline and glutamic acid at position 321 is replaced with aspartic acid, respectively;
   (6) a mutant containing an amino acid sequence represented by SEQ ID NO: 17 in which serine at position 505 in the wild-type hNAGLU amino acid sequence represented by SEQ ID NO: 1 is replaced with alanine and isoleucine at position 506 is replaced with valine; and
   (7) a mutant containing an amino acid sequence represented by SEQ ID NO: 19 in which serine at position 526 in the wild-type hNAGLU amino acid sequence represented by SEQ ID NO: 1 is replaced with asparagine and alanine at position 528 is replaced with threonine, respectively.
2. A hNAGLU mutant obtained by adding a mutation to the hNAGLU mutant containing an amino acid sequence represented by SEQ ID NO: 3 according to 1 above, while glutamic acid at position 36 and serine at position 37 of the amino acid sequence retained, and selected from the group consisting of (1'-a) to (1'-h) below:
   (1'-a) a hNAGLU mutant in which an amino acid residue constituting the amino acid sequence is replaced with another amino acid residue and the number of the replaced amino acid residue is 1 to 10, 1 to 5, or 1 to 3, for example, 1 or 2;
   (1'-b) a hNAGLU mutant in which an amino acid residue constituting the amino acid sequence is deleted and the number of the deleted amino acid residue is 1 to 10, 1 to 5, or 1 to 3, for example, 1 or 2;
   (1'-c) a hNAGLU mutant having both of the replacement in the above 1'-a and the deletion in the above 1'-b;
   (1'-d) a hNAGLU mutant in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
   (1'-e) a hNAGLU mutant having both of the replacement in the above 1'-a and the addition in the above 1'-d;
   (1'-f) a hNAGLU mutant having both of the deletion in the above 1'-b and the addition in the above 1'-d;
   (1'-g) a hNAGLU mutant having all of the replacement in the above 1'-a, the deletion in the above 1'-b, and the addition in the above 1'-d; and
   (1'-h) a hNAGLU mutant showing 80% or more, 85% or more, 90% or more, or 95% or more identity, or 98% or more or 99% identity to the amino acid sequence.
3. A hNAGLU mutant obtained by adding a mutation to the hNAGLU mutant (1) containing an amino acid sequence represented by SEQ ID NO: 5 according to 1 above, while retaining serine at position 45 of the amino acid sequence, and selected from the group consisting of (2'-a) to (2'-h) below:
   (2'-a) a hNAGLU mutant in which an amino acid residue constituting the amino acid sequence is replaced with another amino acid residue and the number of the replaced amino acid residues is 1 to 10, 1 to 5, or 1 to 3, for example, 1 or 2;
   (2'-b) a hNAGLU mutant in which an amino acid residue constituting the amino acid sequence is deleted and the number of the deleted amino acid residue is 1 to 10, 1 to 5, or 1 to 3, for example, 1 or 2;
   (2'-c) a hNAGLU mutant having both of the replacement in the above 2'-a and the deletion in the above 2'-b;
   (2'-d) a hNAGLU mutant in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
   (2'-e) a hNAGLU mutant having both of the replacement in the above 2'-a and addition in the above 2'-d;
   (2'-f) a hNAGLU mutant having both of the deletion in the above 2'-b and the addition in the above 2'-d;
   (2'-g) a hNAGLU mutant having all of the replacement in the above 2'-a, the deletion in the above 2'-b, and the addition in the above 2'-d; and
   (2'-h) a hNAGLU mutant showing 80% or more, 85% or more, 90% or more, or 95% or more identity, or 98% or more or 99% identity to the amino acid sequence.
4. A hNAGLU mutant obtained by adding a mutation to the hNAGLU mutant containing an amino acid sequence represented by SEQ ID NO: 9 according to 1 above, while retaining arginine at position 209 of the amino acid sequence, and selected from the group consisting of (3'-a) to (3'-h) below:
   (3'-a) a hNAGLU mutant in which an amino acid residue constituting the amino acid sequence is replaced with another amino acid residue and the number of the replaced amino acid residue is 1 to 10, 1 to 5, or 1 to 3, for example, 1 or 2;
   (3'-b) a hNAGLU mutant in which an amino acid residue constituting the amino acid sequence is deleted and the number of the deleted amino acid residue is 1 to 10, 1 to 5, or 1 to 3, for example, 1 or 2;
   (3'-c) a hNAGLU mutant having both of the replacement in the above 3'-a and the deletion in the above 3'-b;
   (3'-d) a hNAGLU mutant in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
   (3'-e) a hNAGLU mutant having both of the replacement in the above 3'-a and the addition in the above 3'-d;
   (3'-f) a hNAGLU mutant having both of the deletion in the above 3'-b and the addition in the above 3'-d;
   (3'-g) a hNAGLU mutant having all of the replacement in the above 3'-a, the deletion in the above 3'-b, and the addition in the above 3'-d; and
   (3'-h) a hNAGLU mutant showing 80% or more, 85% or more, 90% or more, or 95% or more identity, or 98% or more or 99% identity to the amino acid sequence.
5. A hNAGLU mutant obtained by adding a mutation to the hNAGLU mutant containing an amino acid sequence represented by SEQ ID NO: 11 according to 1 above, while retaining lysine at position 228 of the amino acid sequence, and selected from the group consisting of (4'-a) to (4'-h) below:
   (4'-a) a hNAGLU mutant in which an amino acid residue constituting the amino acid sequence is replaced with another amino acid residue and the number of the replaced amino acid residue is 1 to 10, 1 to 5, or 1 to 3, for example, 1 or 2;
   (4'-b) a hNAGLU mutant in which an amino acid residue constituting the amino acid sequence is deleted and the number of the deleted amino acid residue is 1 to 10, 1 to 5, or 1 to 3, for example, 1 or 2;
   (4'-c) a hNAGLU mutant having both of the replacement in the above 4'-a and the deletion in the above 4'-b;
   (4'-d) a hNAGLU mutant in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
   (4'-e) a hNAGLU mutant having both of the replacement in the above 4'-a and the addition in the above 4'-d;
   (4'-f) a hNAGLU mutant having both of the deletion in the above 4'-b and the addition in the above 4'-d;
   (4'-g) a hNAGLU mutant having all of the replacement in the above 4'-a, the deletion in the above 4'-b, and the addition in the above 4'-d; and
   (4'-h) a hNAGLU mutant showing 80% or more, 85% or more, 90% or more, or 95% or more identity, or 98% or more or 99% identity to the amino acid sequence.
6. A hNAGLU mutant obtained by adding a mutation to the hNAGLU mutant containing an amino acid sequence represented by SEQ ID NO: 15 according to 1 above, while retaining proline at position 320 and aspartic acid at position 321 of the amino acid sequence, and selected from the group consisting of (5'-a) to (5'-h) below:
   (5'-a) a hNAGLU mutant in which an amino acid residue constituting the amino acid sequence is replaced with another amino acid residue and the number of replaced amino acid residues is 1 to 10, 1 to 5, or 1 to 3, for example, 1 or 2;
   (5'-b) a hNAGLU mutant in which an amino acid residue constituting the amino acid sequence is deleted and the number of the deleted amino acid residue is 1 to 10, 1 to 5, or 1 to 3, for example, 1 or 2;
   (5'-c) a hNAGLU mutant having both of the replacement in the above 5'-a and the deletion in the above 5'-b;
   (5'-d) a hNAGLU mutant in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
   (5'-e) a hNAGLU mutant having both of the replacement in the above 5'-a and the addition in the above 5'-d;
   (5'-f) a hNAGLU mutant having both of the deletion in the above 5'-b and the addition in the above 5'-d;
   (5'-g) a hNAGLU mutant having all of the replacement in the above 5'-a, the deletion in the above 5'-b, and the addition in the above 5'-d; and
   (5'-h) a hNAGLU mutant showing 80% or more, 85% or more, 90% or more, or 95% or more identity, or 98% or more or 99% identity to the amino acid sequence.
7. A hNAGLU mutant obtained by adding a mutation to the hNAGLU mutant containing an amino acid sequence represented by SEQ ID NO: 17 according to 1 above, while retaining alanine at position 505 and valine at position 506 of the amino acid sequence, and selected from the group consisting of (6'-a) to (6'-h) below:
   (6'-a) a hNAGLU mutant in which an amino acid residue constituting the amino acid sequence is replaced with another amino acid residue and the number of the replaced amino acid residue is 1 to 10, 1 to 5, or 1 to 3, for example, 1 or 2;
   (6'-b) a hNAGLU mutant in which an amino acid residue constituting the amino acid sequence is deleted and the number of the deleted amino acid residue is 1 to 10, 1 to 5, or 1 to 3, for example, 1 or 2;
   (6'-c) a hNAGLU mutant having both of the replacement in the above 6'-a and the deletion in the above 6'-b;
   (6'-d) a hNAGLU mutant in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
   (6'-e) a hNAGLU mutant having both of the replacement in the above 6'-a and the addition in the above 6'-d;
   (6'-f) a hNAGLU mutant having both of the deletion in the above 6'-b and the addition in the above 6'-d;
   (6'-g) a hNAGLU mutant having all of the replacement in the above 6'-a, the deletion in the above 6'-b, and the addition in the above 6'-d; and
   (6'-h) a hNAGLU mutant showing 80% or more, 85% or more, 90% or more, or 95% or more identity, or 98% or more or 99% identity to the amino acid sequence.
8. A hNAGLU mutant obtained by adding a mutation to the hNAGLU mutant containing an amino acid sequence represented by SEQ ID NO: 19 according to 1 above, while retaining asparagine at position 526 and threonine at position 528 of the amino acid sequence, and selected from the group consisting of (7'-a) to (7'-h) below:
   (7'-a) a hNAGLU mutant in which an amino acid residue constituting the amino acid sequence is replaced with another amino acid residue and the number of the replaced amino acid residue is 1 to 10, 1 to 5, or 1 to 3, for example, 1 or 2;
   (7'-b) a hNAGLU mutant in which an amino acid residue constituting the amino acid sequence is deleted and the number of the deleted amino acid residue is 1 to 10, 1 to 5, or 1 to 3, for example, 1 or 2;
   (7'-c) a hNAGLU mutant having both of the replacement in the above 7'-a and the deletion in the above 7'-b;
   (7'-d) a hNAGLU mutant in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
   (7'-e) a hNAGLU mutant having both of the replacement in the above 7'-a and the addition in the above 7'-d;
   (7'-f) a hNAGLU mutant having both of the deletion in the above 7'-b and the addition in the above 7'-d;
   (7'-g) a hNAGLU mutant having all of the replacement in the above 7'-a, the deletion in the above 7'-b, and the addition in the above 7'-d; and
   (7'-h) a hNAGLU mutant showing 80% or more, 85% or more, 90% or more, or 95% or more identity, or 98% or more or 99% identity to the amino acid sequence.
9. The hNAGLU mutant selected from the group consisting of (8) to (15) below according to 4 above:
   (8) a hNAGLU mutant containing an amino acid sequence represented by SEQ ID NO: 25, obtained by adding mutations to the hNAGLU mutant containing an amino acid sequence represented by SEQ ID NO: 9, in that mutations lysine at position 36 is replaced with glutamic acid and proline at position 37 is replaced with serine, respectively while arginine at position 209 of the amino acid sequence retained ;
   (9) a hNAGLU mutant containing an amino acid sequence represented by SEQ ID NO: 27, obtained by adding a mutation to the hNAGLU mutant containing an amino acid sequence represented by SEQ ID NO: 9, in that mutation serine is added between leucine at position 44 and glycine at position 45, while arginine at position 209 of the amino acid sequence retained;
   (10) a hNAGLU mutant containing an amino acid sequence represented by SEQ ID NO: 29, obtained by adding mutations to the hNAGLU mutant containing an amino acid sequence represented by SEQ ID NO: 9, in that mutations threonine at position 320 is replaced with proline and glutamic acid at position 321 is replaced with aspartic acid, while arginine at position 209 of the amino acid sequence retained;
   (11) a hNAGLU mutant containing an amino acid sequence represented by SEQ ID NO: 31, obtained by adding mutations to the hNAGLU mutant containing an amino acid sequence represented by SEQ ID NO: 9 in that mutations lysine at position 36 is replaced with glutamic acid, proline at position 37 is replaced with serine, respectively, serine is added between leucine at position 44 and glycine at position 45, while arginine at position 209 of the amino acid sequence retained;
   (12) a hNAGLU mutant containing an amino acid sequence represented by SEQ ID NO: 33, obtained by adding mutations to the hNAGLU mutant containing an amino acid sequence represented by SEQ ID NO: 9 in that mutations valine at position 54 is replaced with isoleucine and arginine at position 620 is replaced with lysine, respectively, while arginine at position 209 of the amino acid sequence retained
   (13) a hNAGLU mutant containing an amino acid sequence represented by SEQ ID NO: 35, obtained by adding mutations to the hNAGLU mutant containing an amino acid sequence represented by SEQ ID NO: 9, in that mutations valine at position 54 is replaced with isoleucine and serine is added between leucine at position 44 and glycine at position 45, while arginine at position 209 of the amino acid sequence retained;
   (14) a hNAGLU mutant containing an amino acid sequence represented by SEQ ID NO: 37, obtained by adding mutations to the hNAGLU mutant containing an amino acid sequence represented by SEQ ID NO: 9, in that mutations arginine at position 620 is replaced with lysine and serine is added between leucine at position 44 and glycine at position 45, while arginine at position 209 of the amino acid sequence retained; and
   (15) a hNAGLU mutant containing an amino acid sequence represented by SEQ ID NO: 39, obtained by adding mutations to the hNAGLU mutant containing an amino acid sequence represented by SEQ ID NO: 9 in that mutations valine at position 54 is replaced with isoleucine, and arginine at position 620 is replaced with lysine, respectively, and serine is added between leucine at position 44 and glycine at position 45, while arginine at position 209 of the amino acid sequence retained.
10. A hNAGLU mutant obtained by adding a mutation to the hNAGLU mutant containing an amino acid sequence represented by SEQ ID NO: 25 according to 9 above, while arginine at position 209, glutamic acid at position 36, and serine at position 37 of the amino acid sequence retained, and selected from the group consisting of (8'-a) to (8'-h) below:
   (8'-a) a hNAGLU mutant in which an amino acid residue constituting the amino acid sequence is replaced with another amino acid residue and the number of the replaced amino acid residue is 1 to 10, 1 to 5, or 1 to 3, for example, 1 or 2;
   (8'-b) a hNAGLU mutant in which an amino acid residue constituting the amino acid sequence is deleted and the number of the deleted amino acid residue is 1 to 10, 1 to 5, or 1 to 3, for example, 1 or 2;
   (8'-c) a hNAGLU mutant having both of the replacement in the above 8'-a and the deletion in the above 8'-b;
   (8'-d) a hNAGLU mutant in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
   (8'-e) a hNAGLU mutant having both of the replacement in the above 8'-a and the addition in the above 8'-d;
   (8'-f) a hNAGLU mutant having both of the deletion in the above 8'-b and the addition in the above 8'-d;
   (8'-g) a hNAGLU mutant having all of the replacement in the above 8'-a, the deletion in the above 8'-b, and the addition in the above 8'-d; and
   (8'-h) a hNAGLU mutant showing 80% or more, 85% or more, 90% or more, or 95% or more identity, or 98% or more or 99% identity to the amino acid sequence.
11. A hNAGLU mutant obtained by adding a mutation to the hNAGLU mutant containing an amino acid sequence represented by SEQ ID NO: 27 according to 9 above, while arginine at position 210 and serine at position 45 of the amino acid sequence retained, and selected from the group consisting of (9'-a) to (9'-h) below:
   (9'-a) a hNAGLU mutant in which an amino acid residue constituting the amino acid sequence is replaced with another amino acid residue and the number of the replaced amino acid residue is 1 to 10, 1 to 5, or 1 to 3, for example, 1 or 2;
   (9'-b) a hNAGLU mutant in which an amino acid residue constituting the amino acid sequence is deleted and the number of the deleted amino acid residue is 1 to 10, 1 to 5, or 1 to 3, for example, 1 or 2;
   (9'-c) a hNAGLU mutant having both of the replacement in the above 9'-a and the deletion in the above 9'-b;
   (9'-d) a hNAGLU mutant in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
   (9'-e) a hNAGLU mutant having both of the replacement in the above 9'-a and the addition in the above 9'-d;
   (9'-f) a hNAGLU mutant having both of the deletion in the above 9'-b and the addition in the above 9'-d;
   (9'-g) a hNAGLU mutant having both of the replacement in the above 9'-a, the deletion in the above 9'-b, and the addition in the above 9'-d; and
   (9'-h) a hNAGLU mutant showing 80% or more, 85% or more, 90% or more, or 95% or more identity, or 98% or more or 99% identity to the amino acid sequence.
12. A hNAGLU mutant obtained by adding a mutation to the hNAGLU mutant containing an amino acid sequence represented by SEQ ID NO: 29 according to 9 above, while arginine at position 209, proline at position 320, and aspartic acid at position 321 of the amino acid sequence retained, and selected from the group consisting of (10'-a) to (10'-h) below:
   (10'-a) a hNAGLU mutant in which an amino acid residue constituting the amino acid sequence is replaced with another amino acid residue and the number of the replaced amino acid residue is 1 to 10, 1 to 5, or 1 to 3, for example, 1 or 2;
   (10'-b) a hNAGLU mutant in which an amino acid residue constituting the amino acid sequence is deleted and the number of the deleted amino acid residue is 1 to 10, 1 to 5, or 1 to 3, for example, 1 or 2;
   (10'-c) a hNAGLU mutant having both of the replacement in the above 10'-a and deletion in the above 10'-b;
   (10'-d) a hNAGLU mutant in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
   (10'-e) a hNAGLU mutant having both of the replacement in the above 10'-a and the addition in the above 10'-d;
   (10'-f) a hNAGLU mutant having both of the deletion in the above 10'-b and the addition in the above 10'-d;
   (10'-g) a hNAGLU mutant having all of the replacement in the above 10'-a, the deletion in the above 10'-b, and the addition in the above 10'-d; and
   (10'-h) a hNAGLU mutant showing 80% or more, 85% or more, 90% or more, or 95% or more identity, or 98% or more or 99% identity to the amino acid sequence.
13. A hNAGLU mutant obtained by adding a mutation to the hNAGLU mutant containing an amino acid sequence represented by SEQ ID NO: 31 according to 9 above, while arginine at position 210, glutamic acid at position 36, serine at position 37, and serine at position 45 of the amino acid sequence retained, and selected from the group consisting of (11'-a) to (11'-h) below:
   (11'-a) a hNAGLU mutant in which an amino acid residue constituting the amino acid sequence is replaced with another amino acid residue and the number of the replaced amino acid residue is 1 to 10, 1 to 5, or 1 to 3, for example, 1 or 2;
   (11'-b) a hNAGLU mutant in which an amino acid residue constituting the amino acid sequence is deleted and the number of the deleted amino acid residue is 1 to 10, 1 to 5, or 1 to 3, for example, 1 or 2;
   (11'-c) a hNAGLU mutant having both of the replacement in the above 11'-a and the deletion in the above 11'-b;
   (11'-d) a hNAGLU mutant in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
   (11'-e) a hNAGLU mutant having both of the replacement in the above 11'-a and the addition in the above 11'-d;
   (11'-f) a hNAGLU mutant having both of the deletion in the above 11'-b and the addition in the above 11'-d;
   (11'-g) a hNAGLU mutant having all of the replacement in the above 11'-a, the deletion in the above 11'-b, and the addition in the above 11'-d; and
   (11'-h) a hNAGLU mutant showing 80% or more, 85% or more, 90% or more, or 95% or more identity, or 98% or more or 99% identity to the amino acid sequence.
14. A hNAGLU mutant obtained by adding a mutation to the hNAGLU mutant containing an amino acid sequence represented by SEQ ID NO: 33 according to 9 above, while arginine at position 209, isoleucine at position 54, and lysine at position 620 of the amino acid sequence retained, and selected from the group consisting of (12'-a) to (12'-h) below:
   (12'-a) a hNAGLU mutant in which an amino acid residue constituting the amino acid sequence is replaced with another amino acid residue and the number of the replaced amino acid residue is 1 to 10, 1 to 5, or 1 to 3, for example, 1 or 2;
   (12'-b) a hNAGLU mutant in which an amino acid residue constituting the amino acid sequence is deleted and the number of the deleted amino acid residue is 1 to 10, 1 to 5, or 1 to 3, for example, 1 or 2;
   (12'-c) a hNAGLU mutant having both of the replacement in the above 12'-a and the deletion in the above 12'-b;
   (12'-d) a hNAGLU mutant in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
   (12'-e) a hNAGLU mutant having both of the replacement in the above 12'-a and the addition in the above 12'-d;
   (12'-f) a hNAGLU mutant having both of the deletion in the above 12'-b and the addition in the above 12'-d;
   (12'-g) a hNAGLU mutant having all of the replacement in the above 12'-a, the deletion in the above 12'-b, and the addition in the above 12'-d; and
   (12'-h) a hNAGLU mutant showing 80% or more, 85% or more, 90% or more, or 95% or more identity, or 98% or more or 99% identity to the amino acid sequence.
15. A hNAGLU mutant obtained by adding a mutation to the hNAGLU mutant containing an amino acid sequence represented by SEQ ID NO: 35 according to 9 above, while arginine at position 210, isoleucine at position 55, and serine at position 45 of the amino acid sequence retained, and selected from the group consisting of (13'-a) to (13'-h) below:
   (13'-a) a hNAGLU mutant in which an amino acid residue constituting the amino acid sequence is replaced with another amino acid residue and the number of the replaced amino acid residue is 1 to 10, 1 to 5, or 1 to 3, for example, 1 or 2;
   (13'-b) a hNAGLU mutant in which an amino acid residue constituting the amino acid sequence is deleted and the number of the deleted amino acid residue is 1 to 10, 1 to 5, or 1 to 3, for example, 1 or 2;
   (13'-c) a hNAGLU mutant having both of the replacement in the above 13'-a and the deletion in the above 13'-b;
   (13'-d) a hNAGLU mutant in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
   (13'-e) a hNAGLU mutant having both of the replacement in the above 13'-a and the addition in the above 13'-d;
   (13'-f) a hNAGLU mutant having both of the deletion in the above 13'-b and the addition in the above 13'-d;
   (13'-g) a hNAGLU mutant having all of the replacement in the above 13'-a, the deletion in the above 13'-b, and the addition in the above 13'-d; and
   (13'-h) a hNAGLU mutant showing 80% or more, 85% or more, 90% or more, or 95% or more identity, or 98% or more or 99% identity to the amino acid sequence.
16. A hNAGLU mutant obtained by adding a mutation to the hNAGLU mutant containing an amino acid sequence represented by SEQ ID NO: 37 according to 9 above, while arginine at position 210, lysine at position 621, and serine at position 45 of the amino acid sequence retained, and selected from the group consisting of (14'-a) to (14'-h) below:
   (14'-a) a hNAGLU mutant in which an amino acid residue constituting the amino acid sequence is replaced with another amino acid residue and the number of the replaced amino acid residue is 1 to 10, 1 to 5, or 1 to 3, for example, 1 or 2;
   (14'-b) a hNAGLU mutant in which an amino acid residue constituting the amino acid sequence is deleted and the number of the deleted amino acid residue is 1 to 10, 1 to 5, or 1 to 3, for example, 1 or 2;
   (14'-c) a hNAGLU mutant having both of the replacement in the above 14'-a and the deletion in the above 14'-b;
   (14'-d) a hNAGLU mutant in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
   (14'-e) a hNAGLU mutant having both of the replacement in the above 14'-a and the addition in the above 14'-d;
   (14'-f) a hNAGLU mutant having both of the deletion in the above 14'-b and the addition in the above 14'-d;
   (14'-g) a hNAGLU mutant having all of the replacement in the above 14'-a, the deletion in the above 14'-b, and the addition in the above 14'-d; and
   (14'-h) a hNAGLU mutant showing 80% or more, 85% or more, 90% or more, or 95% or more identity, or 98% or more or 99% identity to the amino acid sequence.
17. A hNAGLU mutant obtained by adding a mutation to the hNAGLU mutant containing an amino acid sequence represented by SEQ ID NO: 39 according to 9 above, while arginine at position 210, isoleucine at position 55, lysine at position 621, and serine at position 45 of the amino acid sequence retained, and selected from the group consisting of (15'-a) to (15'-h) below:
   (15'-a) a hNAGLU mutant in which an amino acid residue constituting the amino acid sequence is replaced with another amino acid residue and the number of the replaced amino acid residue is 1 to 10, 1 to 5, or 1 to 3, for example, 1 or 2;
   (15'-b) a hNAGLU mutant in which an amino acid residue constituting the amino acid sequence is deleted and the number of the deleted amino acid residue is 1 to 10, 1 to 5, or 1 to 3, for example, 1 or 2;
   (15'-c) a hNAGLU mutant having both of the replacement in the above 15'-a and the deletion in the above 15'-b;
   (15'-d) a hNAGLU mutant in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
   (15'-e) a hNAGLU mutant having both of the replacement in the above 15'-a and the addition in the above 15'-d;
   (15'-f) a hNAGLU mutant having both of the deletion in the above 15'-b and the addition in the above 15'-d;
   (15'-g) a hNAGLU mutant having all of the replacement in the above 15'-a, the deletion in the above 15'-b, and the addition in the above 15'-d; and
   (15'-h) a hNAGLU mutant showing 80% or more, 85% or more, 90% or more, or 95% or more identity, or 98% or more or 99% identity to the amino acid sequence.
18. A DNA including a gene encoding the hNAGLU mutant according to any one of 1 to 17 above.
19. An expression vector including the DNA according to 18 above.
20. A mammalian cell transformed with the expression vector according to 19 above.
21. A method for producing a hNAGLU mutant, including a step of culturing the mammalian cell according to 20 above in a serum-free medium.
22. A fusion protein of the hNAGLU mutant according to any one of 1 to 17 above and an antibody, in which the antibody binds to a receptor on cerebrovascular endothelial cells such that the fusion protein is capable of crossing a blood-brain barrier (BBB).
23. The fusion protein according to 22 above, in which the receptor on cerebrovascular endothelial cells is selected from the group consisting of an insulin receptor, a transferrin receptor, a leptin receptor, a lipoprotein receptor, and an IGF receptor.
24. The fusion protein of 22 above, in which the receptor on cerebrovascular endothelial cells is the transferrin receptor.
25. The fusion protein according to any one of 22 to 24 above, in which the antibody is a Fab antibody, a F(ab')₂ antibody, a F(ab') antibody, a single domain antibody, a single chain antibody, or an Fc antibody.
26. The fusion protein according to any one of 22 to 25 above, in which the hNAGLU mutant binds to either the C-terminus side or the N-terminus side of a light chain of the antibody.
27. The fusion protein according to any one of 22 to 25 above, in which the hNAGLU mutant binds to either the C-terminus side or the N-terminus side of a heavy chain of the antibody.
28. The fusion protein according to any one of 22 to 27 above, in which the hNAGLU mutant binds to either the C-terminus side or the N-terminus side of the light chain, or either the C-terminus side or the N-terminus side of the heavy chain of the antibody, via a linker sequence.
29. The fusion protein according to 28 above, in which the linker sequence consists of 1 to 50 amino acid residues.
30. The fusion protein according to 29 above, in which the linker sequence includes an amino acid sequence selected from the group consisting of one glycine, one serine, an amino acid sequence Gly-Ser, an amino acid sequence Ser-Ser, an amino acid sequence Gly-Gly-Ser, an amino acid sequence represented by SEQ ID NO: 3, an amino acid sequence represented by SEQ ID NO: 4, an amino acid sequence represented by SEQ ID NO: 5, and an amino acid sequence consisting of 1 to 10 of consecutively-linked aforementioned amino acid sequences.
31. A DNA including a gene encoding the fusion protein according to any one of 22 to 30 above.
32. An expression vector including the DNA according to 31 above.
33. A mammalian cell transformed with the expression vector according to 32 above.
34. A method for producing a fusion protein of a hNAGLU mutant and an antibody, including a step of culturing the mammalian cell according to 33 above in a serum-free medium.

### Advantageous Effects of Invention

According to the present invention, hNAGLU, which can be administered as enzyme replacement therapy for the treatment of patients with mucopolysaccharidosis type IIIB, can be efficiently produced using gene recombination technology.

### Brief Description of Drawings

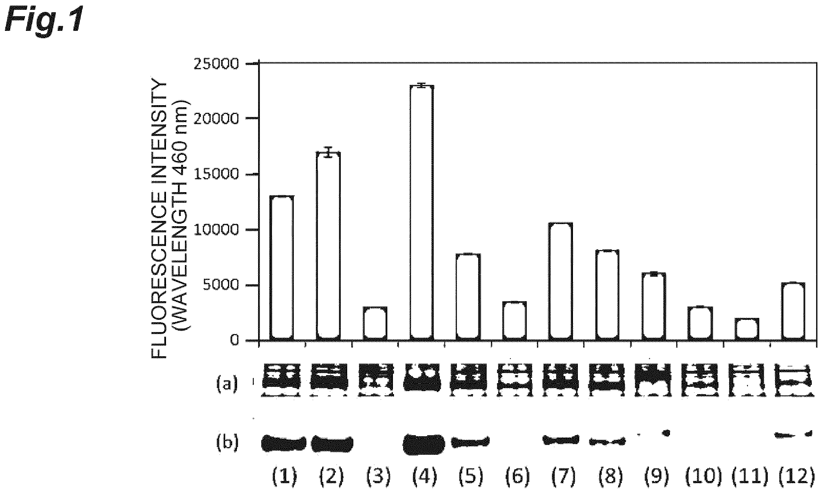

FIG. 1 is a diagram showing results of an experiment to confirm an expression level of hNAGLU mutants by transient expression (Example 6). The vertical axis of the bar graph indicates fluorescence intensity. FIG. 1(a) shows SDS-page patterns of bands corresponding to wild-type hNAGLU or hNAGLU mutants, and FIG. 1(b) shows Western blot patterns of bands corresponding to wild-type hNAGLU or hNAGLU mutants, respectively. FIG. 1(1) indicates expression level data for a K36E/P37S hNAGLU mutant, FIG. 1(2) indicates expression level data for an L44_G45insS hNAGLU mutant, FIG. 1(3) indicates expression level data for an R129Q hNAGLU mutant, FIG. 1(4) indicates expression level data for a Q209R hNAGLU mutant, FIG. 1(5) indicates expression level data for an E228K hNAGLU mutant, FIG. 1(6) indicates expression level data for a T240V hNAGLU mutant, FIG. 1(7) indicates expression level data for a T320P/E321D hNAGLU mutant, FIG. 1(8) indicates expression level data for an S505A/I506V hNAGLU mutant, FIG. 1(9) indicates expression level data for an S526N/A528T hNAGLU mutant, FIG. 1(10) indicates expression level data for a D613Q hNAGLU mutant, FIG. 1(11) indicates expression level data for an H204K hNAGLU mutant, and FIG. 1(12) indicates expression level data for a wild-type hNAGLU, respectively.

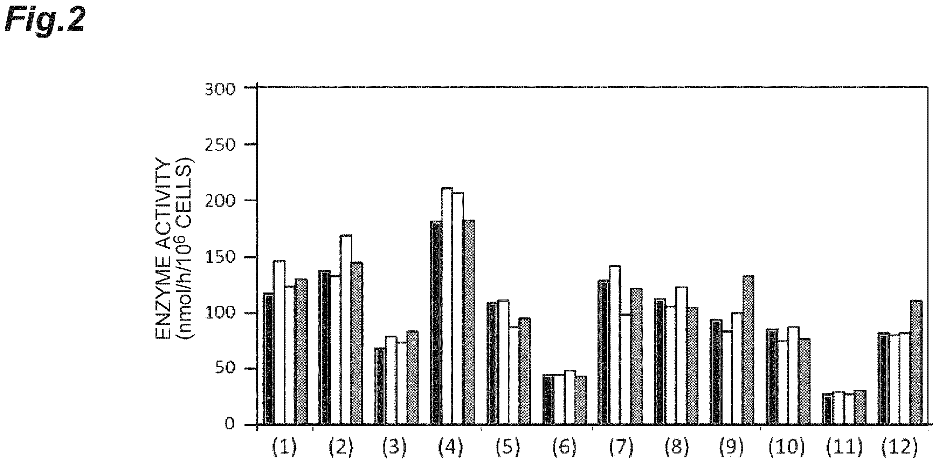

FIG. 2 is a diagram showing results of an experiment to confirm an expression level of hNAGLU mutants using bulk cells (Example 10). The vertical axis of the bar graph indicates an enzyme activity level of hNAGLU expressed by 1 × 10⁶ cells (nmol/h/10⁶ cells) for each of hNAGLU mutants. FIG. 2(1) indicates expression level data for a K36E/P37S hNAGLU mutant, FIG. 2(2) indicates expression level data for an L44_G45insS hNAGLU mutant, FIG. 2(3) indicates expression level data for an R129Q hNAGLU mutant, FIG. 2(4) indicates expression level data for a Q209R hNAGLU mutant, FIG. 2(5) indicates expression level data for an E228K hNAGLU mutant, FIG. 2(6) indicates expression level data for a T240V hNAGLU mutant, FIG. 2(7) indicates expression level data for a T320P/E321D hNAGLU mutant, FIG. 2(8) indicates expression level data for an S505A/I506V hNAGLU mutant, FIG. 2(9) indicates expression level data for an S526N/A528T hNAGLU mutant, FIG. 2(10) indicates expression level data for a D613Q hNAGLU mutant, FIG. 2(11) indicates expression level data for an H204K hNAGLU mutant, and FIG. 2(12) indicates expression level data for wild-type hNAGLU, respectively. Measurements were repeated four times for each of FIGS. 2(1) to 2(4), and each measurement value is shown in the drawing.

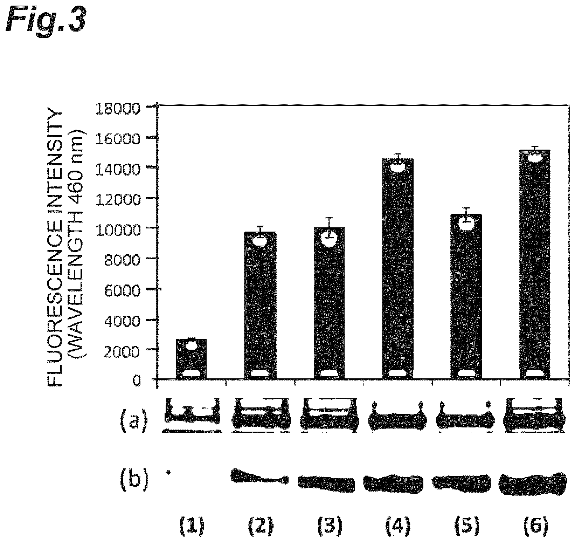

FIG. 3 is a diagram showing results of an experiment to confirm an expression level of hNAGLU mutants by transient expression (Example 16). The vertical axis of the bar graph indicates fluorescence intensity. FIG. 3(a) shows SDS-page patterns of bands corresponding to wild-type hNAGLU or hNAGLU mutants, and FIG. 3(b) shows Western blot patterns of bands corresponding to wild-type hNAGLU or hNAGLU mutants, respectively. FIG. 3(1) indicates expression level data for wild-type hNAGLU, FIG. 3(2) indicates expression level data for a Q209R hNAGLU mutant, FIG. 3(3) indicates expression level data for a K36E/P37S/Q209R hNAGLU mutant, FIG. 3(4) indicates expression level data for an L44_G45insS/Q209R hNAGLU mutant, FIG. 3(5) indicates expression level data for Q209R/T320P/E321D hNAGLU mutant, and FIG. 3(6) indicates expression level data for a K36E/P37S/L44_G45insS/Q209R hNAGLU mutant, respectively.

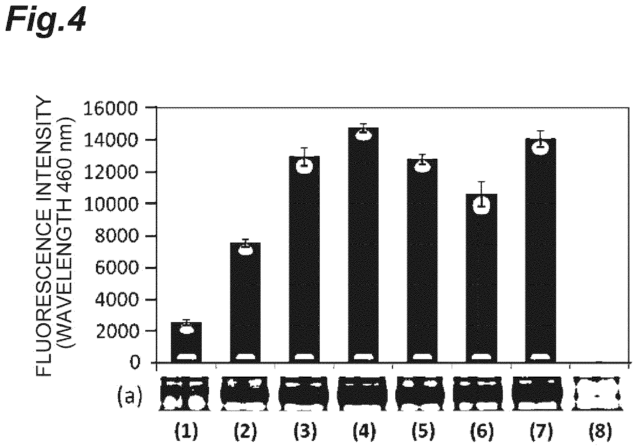

FIG. 4 is a diagram showing results of an experiment to confirm an expression level of hNAGLU mutants by transient expression (Example 16). The vertical axis of the bar graph indicates fluorescence intensity. FIG. 4(a) shows SDS-page patterns of bands corresponding to wild-type hNAGLU or hNAGLU mutants. FIG. 4(1) indicates data of expression level for wild-type hNAGLU, FIG. 4(2) indicates data of expression level for a Q209R hNAGLU mutant, FIG. 4(3) indicates data of expression level for an L44_G45insS/Q209R hNAGLU mutant, FIG. 4(4) indicates data of expression level for a V54I/Q209R/R620K hNAGLU mutant, FIG. 4(5) indicates data of expression level for an L44_G45insS/V54I/Q209R hNAGLU mutant, FIG. 4(6) indicates data of expression level for an L44_G45insS/Q209R/R620K hNAGLU mutant, FIG. 4(7) indicates data of expression level for an L44_G45insS/V54I/Q209R/R620K hNAGLU mutant, and FIG. 4(8) indicates data of expression level for a negative control.

### Description of Embodiments

In the present specification, when simply referred to as "human α-N-acetylglucosaminidase" or "hNAGLU," this term also includes, without particular distinction, a hNAGLU mutant corresponding to one obtained by performing replacement, deletion, and/or addition (in the present specification, "addition" of amino acid residues means adding residues to the terminus or interior of a sequence) of one or more amino acid residues with respect to an amino acid sequence represented by SEQ ID NO: 1 as long as a function as normal wild-type hNAGLU, such as enzyme activity capable of decomposing heparan sulfate, is retained, in addition to normal wild-type hNAGLU consisting of 720 amino acid residues represented by SEQ ID NO: 1. Wild-type hNAGLU is encoded by a gene having a nucleic acid sequence represented by SEQ ID NO: 2, for example. When replacing an amino acid residue with another amino acid residue, the number of the amino acid residue to be replaced is 1 to 10, 1 to 5, or 1 to 3, for example, 1 or 2. When deleting an amino acid residue, the number of the amino acid residue to be deleted is 1 to 10, 1 to 5, or 1 to 3, for example, 1 or 2. In addition, when deleting an amino acid residue, the N-terminus amino acid residue may be deleted and the number of the amino acid residue to be deleted in that case is 1 to 10, 1 to 5, or 1 to 3, for example, 1 or 2. Moreover, replacement and deletion of these amino acid residues may be combined.

When adding an amino acid residue to the amino acid sequence represented by SEQ ID NO: 1, one or more amino acid residues are added into the hNAGLU amino acid sequence or to the N-terminus side or the C-terminus side of the amino acid sequence. The number of an amino acid residue added at this time is 1 to 10, 1 to 5, or 1 to 3, for example, 1 or 2. In addition, addition and replacement of amino acid residues may be combined, addition and deletion of amino acid residues may be combined, and addition, replacement, and deletion of amino acid residues may be combined. Normal wild-type hNAGLU is biosynthesized as a precursor consisting of 743 amino acid residues, and the leader peptide represented by SEQ ID NO: 67 and consisting of 23 amino acid residues from the N-terminus is removed to become hNAGLU. When an amino acid is added to the N-terminus side of hNAGLU, the amino acid may be derived from the leader peptide. In that case, the amino acid added to the N-terminus is Gly when one amino acid is added, Ala-Gly when two amino acids are added, and Ala-Ala-Gly when two amino acids are added.

A hNAGLU mutant introduced by combining at least two types of mutations among the three types of mutations, such as, replacement, deletion, and addition of amino acids, include (i) to (iv) below, but is not limited thereto:
(i) a hNAGLU mutant containing an amino acid sequence obtained by performing deletion of 0 to 10 amino acid residues, replacement of 0 to 10 amino acid residues with other amino acid residues, and further addition of 0 to 10 amino acid residues, with respect to the amino acid sequence represented by SEQ ID NO: 1;
(ii) a hNAGLU mutant containing an amino acid sequence obtained by performing deletion of 0 to 5 amino acid residues, replacement of 0 to 5 amino acid residues with other amino acid residues, and further addition of 0 to 5 amino acid residues, with respect to the amino acid sequence represented by SEQ ID NO: 1;
(iii) a hNAGLU mutant containing an amino acid sequence obtained by performing deletion of 0 to 3 amino acid residues, replacement of 0 to 3 amino acid residues with other amino acid residues, and further addition of 0 to 3 amino acid residues, with respect to the amino acid sequence represented by SEQ ID NO: 1; and
(iv) a hNAGLU mutant containing an amino acid sequence obtained by performing deletion of 0 to 2 amino acid residues, replacement of 0 to 2 amino acid residues with other amino acid residues, and further addition of 0 to 2 amino acid residues, with respect to the amino acid sequence represented by SEQ ID NO: 1.

The above wild-type or hNAGLU mutant of which a constituent amino acid is modified with sugar chain is also hNAGLU. In addition, the above wild-type or hNAGLU mutant of which a constituent amino acid is modified with phosphoric acid is also hNAGLU. Further, the hNAGLU modified with a substance other than sugar chains and phosphoric acid is also hNAGLU. In addition, the above wild-type or hNAGLU mutant in which the side chain of the constituent amino acid is converted by a replacement reaction or the like is also hNAGLU. Such a conversion includes, but is not limited to, a conversion of a cysteine residue to a formylglycine.

In other words, hNAGLU modified with a sugar chain is included in hNAGLU having the amino acid sequence before modification. In addition, hNAGLU modified with phosphoric acid is also included in hNAGLU having the original amino acid sequence before modification with phosphoric acid. Further, hNAGLU modified with a substance other than sugar chains and phosphoric acid is also included in hNAGLU that has the original amino acid sequence before the modification. In addition, hNAGLU in which a side chain of an amino acid constituting hNAGLU is converted by replacement reaction or the like is also included in hNAGLU having the original amino acid sequence before the conversion. Such a conversion includes, but is not limited to, conversion of a cysteine residue to a formylglycine.

In the present invention, the term "human α-N-acetylglucosaminidase mutant" (hNAGLU mutant) refers to a mutant obtained by performing replacement, deletion, and/or addition (in the present specification, "addition" of amino acid residues means adding residues to the terminus or interior a sequence) of one or more of amino acid residues with respect to the amino acid sequence of normal wild-type hNAGLU (amino acid sequence represented by SEQ ID NO: 1), and having a function as normal wild-type hNAGLU, such as enzyme activity capable of decomposing heparan sulfate. Preferable hNAGLU mutants in the present invention include mutants in which one or more amino acid residues are replaced with other amino acid residues, deleted, or added with respect to the amino acid sequence represented by SEQ ID NO: 1. When replacing an amino acid residue in the amino acid sequence with another amino acid residue, the number of the amino acid residue to be replaced is 1 to 10, 1 to 5, or 1 to 3, for example, 1 or 2. When deleting an amino acid residue, the number of the amino acid residue to be deleted is 1 to 10, 1 to 5, or 1 to 3, for example, 1 or 2. In addition, the hNAGLU mutant may be a combination of replacement and deletion of these amino acid residues.

When adding an amino acid residue to the amino acid sequence represented by SEQ ID NO: 1, one or more amino acid residues are added into the hNAGLU amino acid sequence or to the N-terminus side or the C-terminus side of the amino acid sequence. The number of the amino acid residue added at this time is 1 to 10, 1 to 5, or 1 to 3, for example, 1 or 2. In addition, addition and replacement of amino acid residues may be combined, addition and deletion of amino acid residues may be combined, and addition, replacement, and deletion of amino acid residues may be combined. That is, the hNAGLU mutant is a mutant obtained by introducing a combination of at least two types of mutations out of three types of mutations, that is, replacement, deletion, and addition of amino acid, into the amino acid sequence represented by SEQ ID NO: 1.

A hNAGLU mutant introduced by combining at least two types of mutations among the three types of mutations, such as, replacement, deletion, and addition of amino acids, include (i) to (iv) below, but is not limited thereto:
(i) a hNAGLU mutant containing an amino acid sequence obtained by performing deletion of 0 to 10 amino acid residues, replacement of 0 to 10 amino acid residues with other amino acid residues, and further addition of 0 to 10 amino acid residues, with respect to the amino acid sequence represented by SEQ ID NO: 1 (here, excluding wild-type hNAGLU);
(ii) a hNAGLU mutant containing an amino acid sequence obtained by performing deletion of 0 to 5 amino acid residues, replacement of 0 to 5 amino acid residues with other amino acid residues, and further addition of 0 to 5 amino acid residues, with respect to the amino acid sequence represented by SEQ ID NO: 1 (here, excluding wild-type hNAGLU);
(iii) a hNAGLU mutant containing an amino acid sequence obtained by performing deletion of 0 to 3 amino acid residues, replacement of 0 to 3 amino acid residues with other amino acid residues, and further addition of 0 to 3 amino acid residues, with respect to the amino acid sequence represented by SEQ ID NO: 1 (here, excluding wild-type hNAGLU); and
(iv) a hNAGLU mutant containing an amino acid sequence obtained by performing deletion of 0 to 2 amino acid residues, replacement of 0 to 2 amino acid residues with other amino acid residues, and further addition of 0 to 2 amino acid residues, with respect to the amino acid sequence represented by SEQ ID NO: 1 (here, excluding wild-type hNAGLU).

The position of each mutation and its form (deletion, replacement, and addition) in various hNAGLU mutants compared to normal wild-type hNAGLU can be easily confirmed by alignment of the amino acid sequences of both hNAGLU.

The amino acid sequence of the hNAGLU mutant preferably exhibits 80% or more identity, 85% or more identity, 90% or more identity, or 95% or more identity, for example, 98% or more or 99% identity to the amino acid sequence of the normal wild-type hNAGLU represented by SEQ ID NO: 1.

The identity between the wild-type hNAGLU amino acid sequence and the hNAGLU mutant amino acid sequence can be easily calculated using a well-known homology calculation algorithm. Examples of such algorithms include BLAST (Altschul SF. J Mol. Biol. 215. 403-10, (1990)), Pearson and Lipman's similarity search method (Proc. Natl. Acad. Sci. USA. 85. 2444 (1988)), Smith and Waterman's local homology algorithm (Adv. Appl. Math. 2. 482-9 (1981)).

A replacement of an amino acid in the wild-type hNAGLU amino acid sequence or hNAGLU mutants with another amino acid is, for example, that occurring within families of amino acids that are related in their side chains and chemical properties. Replacements within families of amino acids are expected not to cause major changes in the function of the original protein (that is, conservative amino acid replacements). Examples of such families of amino acids include those shown in (1) to (12) below:
(1) aspartic acid and glutamic acid which are acidic amino acids;
(2) histidine, lysine, and arginine which are basic amino acids;
(3) phenylalanine, tyrosine, and tryptophane which are aromatic amine acids;
(4) serine and threonine which are amino acids having a hydroxyl group (hydroxyamino acids);
(5) methionine, alanine, valine, leucine, and isoleucine which are hydrophobic amino acids;
(6) cysteine, serine, threonine, asparagine, and glutamine which are neutral hydrophilic amino acids;
(7) glycine and proline which are amino acids and affect the orientation of the peptide chain;
(8) asparagine and glutamine which are amide-type amino acids (polar amino acids);
(9) alanine, leucine, isoleucine, and valine which are aliphatic amino acids;
(10) alanine, glycine, serine, and threonine which are amino acids with small side chains;
(11) alanine and glycine which are amino acids with particularly small side chains; and
(12) valine, leucine, and isoleucine which are amino acids with branched chains.

In one embodiment of the present invention, a high-expression type hNAGLU mutant is referred to as a hNAGLU mutant in which an expression level of at least 1.1 times or more, 1.5 times or more, 2 times or more, 4 times or more, 5 times or more, or 6 times or more, for example, 1.5 to 4 times, 2 to 5 times, and 2 to 8 times is achieved when expressed in host cells as a recombinant protein, compared with wild-type hNAGLU expressed in host cells as a recombinant protein under the same conditions. Here, "under the same conditions" refers to that the expression vectors, host cells, culture conditions, and the like are the same.

Preferable embodiments of such a high-expression type hNAGLU mutant include the following (1) to (7):
(1) a hNAGLU mutant containing an amino acid sequence represented by SEQ ID NO: 3 in which lysine at position 36 is replaced with glutamic acid and proline at position 37 is replaced with serine in the wild-type hNAGLU amino acid sequence represented by SEQ ID NO: 1, respectively;
(2) a hNAGLU mutant containing an amino acid sequence represented by SEQ ID NO: 5 in which serine is added between leucine at position 44 and glycine at position 45 in the wild-type hNAGLU amino acid sequence represented by SEQ ID NO: 1;
(3) a hNAGLU mutant containing an amino acid sequence represented by SEQ ID NO: 9 in which glutamine at position 209 in the wild-type hNAGLU amino acid sequence represented by SEQ ID NO: 1 is replaced with arginine;
(4) a hNAGLU mutant containing an amino acid sequence represented by SEQ ID NO: 11 in which glutamic acid at position 228 in the wild-type hNAGLU amino acid sequence represented by SEQ ID NO: 1 is replaced with lysine;
(5) a hNAGLU mutant containing an amino acid sequence represented by SEQ ID NO: 15 in which threonine at position 320 is replaced with proline and glutamic acid at position 321 is replaced with aspartic acid in the wild-type hNAGLU amino acid sequence represented by SEQ ID NO: 1, respectively;
(6) a hNAGLU mutant containing an amino acid sequence represented by SEQ ID NO: 17 in which serine at position 505 is replaced with alanine and isoleucine at position 506 is replaced with valine in the wild-type hNAGLU amino acid sequence represented by SEQ ID NO: 1, respectively; and
(7) a hNAGLU mutant containing an amino acid sequence represented by SEQ ID NO: 19 in which serine at position 526 is replaced with asparagine and alanine at position 528 is replaced with threonine in the wild-type hNAGLU amino acid sequence represented by SEQ ID NO: 1, respectively.

Furthermore, preferable embodiments of such high-expression type hNAGLU mutants include the following (1') to (7').
(1') a hNAGLU mutant, without adding a mutation to glutamic acid at position 36 and serine at position 37 of the amino acid sequence represented by SEQ ID NO: 3:
   (1'-a) in which an amino acid residue constituting the amino acid sequence is replaced with another amino acid residue, and the number of the replaced amino acid residue is 1 to 10, 1 to 5, or 1 to 3, for example, 1 or 2;
   (1'-b) in which an amino acid residue constituting the amino acid sequence is deleted and the number of the deleted amino acid residue is 1 to 10, 1 to 5, or 1 to 3, for example, 1 or 2;
   (1'-c) in which replacement in the above 1'-a and deletion in the above 1'-b are combined;
   (1'-d) in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
   (1'-e) in which replacement in the above 1'-a and addition in the above 1'-d are combined;
   (1'-f) in which deletion in the above 1'-b and addition in the above 1'-d are combined;
   (1'-g) in which replacement in the above 1'-a, deletion in the above 1'-b, and addition in the above 1'-d are combined; and
   (1'-h) showing 80% or more, 85% or more, 90% or more, or 95% or more identity, or 98% or more or 99% identity to the amino acid sequence.
(2') a hNAGLU mutant, without adding a mutation to serine at position 45 of the amino acid sequence represented by SEQ ID NO: 5:
   (2'-a) in which amino an acid residue constituting the amino acid sequence is replaced with another amino acid residue and the number of the replaced amino acid residue is 1 to 10, 1 to 5, or 1 to 3, for example, 1 or 2;
   (2'-b) in which amino an acid residue constituting the amino acid sequence is deleted and the number of the deleted amino acid residue is 1 to 10, 1 to 5, or 1 to 3, for example, 1 or 2;
   (2'-c) which replacement in the above 2'-a and deletion in the above 2'-b are combined;
   (2'-d) in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
   (2'-e) in which replacement in the above 2'-a and addition in the above 2'-d are combined;
   (2'-f) in which deletion in the above 2'-b and addition in the above 2'-d are combined;
   (2'-g) in which replacement in the above 2'-a, deletion in the above 2'-b, and addition in the above 2'-d are combined; and
   (2'-h) showing 80% or more, 85% or more, 90% or more, or 95% or more identity, or 98% or more or 99% identity to the amino acid sequence.
(3') a hNAGLU mutant, without adding a mutation to arginine at position 209 of the amino acid sequence represented by SEQ ID NO: 9:
   (3'-a) in which an amino acid residue constituting the amino acid sequence is replaced with another amino acid residue and the number of the replaced amino acid residue is 1 to 10, 1 to 5, or 1 to 3, for example, 1 or 2;
   (3'-b) in which an amino acid residue constituting the amino acid sequence is deleted and the number of the deleted amino acid residues is 1 to 10, 1 to 5, or 1 to 3, for example, 1 or 2;
   (3'-c) in which replacement in the above 3'-a and deletion in the above 3'-b are combined;
   (3'-d) in which one or more amino acid residues are added into the amino acid sequence or to an N-terminus side or a C-terminus side of the amino acid sequence and the number of amino acid residues added is 1 to 10, 1 to 5, or 1 to 3, for example, 1 or 2;
   (3'-e) in which replacement in the above 3'-a and addition in the above 3'-d are combined;
   (3'-f) in which deletion in the above 3'-b and addition in the above 3'-d are combined;
   (3'-g) in which replacement in the above 3'-a, deletion in the above 3'-b, and addition in the above 3'-d are combined; and
   (3'-h) showing 80% or more, 85% or more, 90% or more, or 95% or more identity, or 98% or more or 99% identity to the amino acid sequence.
(4') a hNAGLU mutant, without adding a mutation to lysine at position 228 of the amino acid sequence represented by SEQ ID NO: 11:
   (4'-a) in which an amino acid residue constituting the amino acid sequence is replaced with another amino acid residue and the number of the replaced amino acid residue is 1 to 10, 1 to 5, or 1 to 3, for example, 1 or 2;
   (4'-b) in which an amino acid residue constituting the amino acid sequence is deleted and the number of the deleted amino acid residue is 1 to 10, 1 to 5, or 1 to 3, for example, 1 or 2;
   (4'-c) in which replacement in the above 4'-a and deletion in the above 4'-b are combined;
   (4'-d) in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
   (4'-e) in which replacement in the above 4'-a and addition in the above 4'-d are combined;
   (4'-f) in which deletion in the above 4'-b and addition in the above 4'-d are combined;
   (4'-g) in which replacement in the above 4'-a, deletion in the above 4'-b, and addition in the above 4'-d are combined; and
   (4'-h) showing 80% or more, 85% or more, 90% or more, or 95% or more identity, or 98% or more or 99% identity to the amino acid sequence.
(5') a hNAGLU mutant, without adding a mutation to proline at position 320 and aspartic acid at position 321 of the amino acid sequence represented by SEQ ID NO: 15:
   (5'-a) in which an amino acid residue constituting the amino acid sequence is replaced with another amino acid residue and the number of the replaced amino acid residue is 1 to 10, 1 to 5, or 1 to 3, for example, 1 or 2;
   (5'-b) in which an amino acid residue constituting the amino acid sequence is deleted and the number of deleted amino acid residue is 1 to 10, 1 to 5, or 1 to 3, for example, 1 or 2;
   (5'-c) in which replacement in the above 5'-a and deletion in the above 5'-b are combined;
   (5'-d) in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
   (5'-e) in which replacement in the above 5'-a and addition in the above 5'-d are combined;
   (5'-f) in which deletion in the above 5'-b and addition in the above 5'-d are combined;
   (5'-g) in which replacement in the above 5'-a, deletion in the above 5'-b, and addition in the above 5'-d are combined; and
   (5'-h) showing 80% or more, 85% or more, 90% or more, or 95% or more identity, or 98% or more or 99% identity to the amino acid sequence.
(6') a hNAGLU mutant, without adding a mutation to alanine at position 505 and valine at position 506 of the amino acid sequence represented by SEQ ID NO: 17:
   (6'-a) in which an amino acid residue constituting the amino acid sequence is replaced with another amino acid residue and the number of the replaced amino acid residue is 1 to 10, 1 to 5, or 1 to 3, for example, 1 or 2;
   (6'-b) in which an amino acid residue constituting the amino acid sequence is deleted and the number of the deleted amino acid residue is 1 to 10, 1 to 5, or 1 to 3, for example, 1 or 2;
   (6'-c) in which replacement in the above 6'-a and deletion in the above 6'-b are combined;
   (6'-d) in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
   (6'-e) in which replacement in the above 6'-a and addition in the above 6'-d are combined;
   (6'-f) in which deletion in the above 6'-b and addition in the above 6'-d are combined;
   (6'-g) in which replacement in the above 6'-a, deletion in the above 6'-b, and addition in the above 6'-d are combined; and
   (6'-h) showing 80% or more, 85% or more, 90% or more, or 95% or more identity, or 98% or more or 99% identity to the amino acid sequence.
(7') a hNAGLU mutant, without adding a mutation to asparagine at position 526 and threonine at position 528 of the amino acid sequence represented by SEQ ID NO: 19:
   (7'-a) in which an amino acid residue constituting the amino acid sequence is replaced with another amino acid residue and the number of the replaced amino acid residue is 1 to 10, 1 to 5, or 1 to 3, for example, 1 or 2;
   (7'-b) in which an amino acid residue constituting the amino acid sequence is deleted and the number of the deleted amino acid residue is 1 to 10, 1 to 5, or 1 to 3, for example, 1 or 2;
   (7'-c) in which replacement in the above 7'-a and deletion in the above 7'-b are combined;
   (7'-d) in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
   (7'-e) in which replacement in the above 7'-a and addition in the above 7'-d are combined;
   (7'-f) in which deletion in the above 7'-b and addition in the above 7'-d are combined;
   (7'-g) in which replacement in the above 7'-a, deletion in the above 7'-b, and addition in the above 7'-d are combined; and
   (7'-h) showing 80% or more, 85% or more, 90% or more, or 95% or more identity, or 98% or more or 99% identity to the amino acid sequence.

Specific embodiments of the high-expression type hNAGLU mutant obtained by further introducing a mutation to the high-expression type hNAGLU mutant containing an amino acid sequence represented by SEQ ID NO: 9 in which glutamine at position 209 in the wild-type hNAGLU amino acid sequence represented by SEQ ID NO: 1 of (1) above is replaced with arginine include the following (8) to (15):
(8) a hNAGLU mutant containing the amino acid sequence represented by SEQ ID NO: 25 in which glutamine at position 209 is replaced with arginine, lysine at position 36 is replaced with glutamic acid, and proline at position 37 is replaced with serine in the wild-type hNAGLU amino acid sequence represented by SEQ ID NO: 1, respectively;
(9) a hNAGLU mutant containing an amino acid sequence represented by SEQ ID NO: 27 in which glutamine at position 209 is replaced with arginine, and serine is added between leucine at position 44 and glycine at position 45 in the wild-type hNAGLU amino acid sequence represented by SEQ ID NO: 1;
(10) a hNAGLU mutant containing an amino acid sequence represented by SEQ ID NO: 29 in which glutamine at position 209 is replaced with arginine, threonine at position 320 is replaced with proline, and glutamic acid at position 321 is replaced with aspartic acid in the wild-type hNAGLU amino acid sequence represented by SEQ ID NO: 1, respectively;
(11) a hNAGLU mutant containing an amino acid sequence represented by SEQ ID NO: 31 in which glutamine at position 209 is replaced with arginine, lysine at position 36 is replaced with glutamic acid, and proline at position 37 is replaced with serine, respectively, and serine is added between leucine at position 44 and glycine at position 45 in the wild-type hNAGLU amino acid sequence represented by SEQ ID NO: 1;
(12) a hNAGLU mutant containing an amino acid sequence represented by SEQ ID NO: 33 in which glutamine at position 209 is replaced with arginine, valine at position 54 is replaced with isoleucine, and arginine at position 620 is replaced with lysine in the wild-type hNAGLU amino acid sequence represented by SEQ ID NO: 1, respectively;
(13) a hNAGLU mutant containing an amino acid sequence represented by SEQ ID NO: 35 in which glutamine at position 209 is replaced with arginine, valine at position 54 is replaced with isoleucine, and serine is added between leucine at position 44 and glycine at position 45 in the wild-type hNAGLU amino acid sequence represented by SEQ ID NO: 1;
(14) a hNAGLU mutant containing an amino acid sequence represented by SEQ ID NO: 37 in which glutamine at position 209 is replaced with arginine, and arginine at position 620 is replaced with lysine, respectively, and serine is added between leucine at position 44 and glycine at position 45 in the wild-type hNAGLU amino acid sequence represented by SEQ ID NO: 1; and
(15) a hNAGLU mutant containing an amino acid sequence represented by SEQ ID NO: 39 in which glutamine at position 209 is replaced with arginine, valine at position 54 is replaced with isoleucine, and arginine at position 620 is replaced with lysine, respectively, and serine is added between leucine at position 44 and glycine at position 45 in the wild-type hNAGLU amino acid sequence represented by SEQ ID NO: 1.

Furthermore, preferable embodiments of such high-expression type hNAGLU mutants include the following (8') to (15').

(8') a hNAGLU mutant, without adding a mutation to arginine at position 209, glutamic acid at position 36, and serine at position 37 of the amino acid sequence represented by SEQ ID NO: 25:
(8'-a) in which an amino acid residue constituting the amino acid sequence is replaced with another amino acid residue and the number of the replaced amino acid residue is 1 to 10, 1 to 5, or 1 to 3, for example, 1 or 2;
(8'-b) in which an amino acid residue constituting the amino acid sequence is deleted and the number of the deleted amino acid residue is 1 to 10, 1 to 5, or 1 to 3, for example, 1 or 2;
(8'-c) in which replacement in the above 8'-a and deletion in the above 8'-b are combined;
(8'-d) in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
(8'-e) in which replacement in the above 8'-a and addition in the above 8'-d are combined;
(8'-f) in which deletion in the above 8'-b and addition in the above 8'-d are combined;
(8'-g) in which replacement in the above 8'-a, deletion in the above 8'-b, and addition in the above 8'-d are combined; and
(8'-h) showing 80% or more, 85% or more, 90% or more, or 95% or more identity, or 98% or more or 99% identity to the amino acid sequence.

(9') a hNAGLU mutant, without adding a mutation to arginine at position 210 and serine at position 45 of the amino acid sequence represented by SEQ ID NO: 27:
(9'-a) in which an amino acid residue constituting the amino acid sequence is replaced with another amino acid residue and the number of the replaced amino acid residue is 1 to 10, 1 to 5, or 1 to 3, for example, 1 or 2;
(9'-b) in which an amino acid residue constituting the amino acid sequence is deleted and the number of the deleted amino acid residue is 1 to 10, 1 to 5, or 1 to 3, for example, 1 or 2;
(9'-c) in which replacement in the above 9'-a and deletion in the above 9'-b are combined;
(9'-d) in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
(9'-e) in which replacement in the above 9'-a and addition in the above 9'-d are combined;
(9'-f) in which deletion in the above 9'-b and addition in the above 9'-d are combined;
(9'-g) in which replacement in the above 9'-a, deletion in the above 9'-b, and addition in the above 9'-d are combined; and
(9'-h) showing 80% or more, 85% or more, 90% or more, or 95% or more identity, or 98% or more or 99% identity to the amino acid sequence.

(10') a hNAGLU mutant, without adding a mutation to arginine at position 209, proline at position 320, and aspartic acid at position 321 of the amino acid sequence represented by SEQ ID NO: 29:
(10'-a) in which an amino acid residue constituting the amino acid sequence is replaced with another amino acid residue and the number of the replaced amino acid residue is 1 to 10, 1 to 5, or 1 to 3, for example, 1 or 2;
(10'-b) in which an amino acid residue constituting the amino acid sequence is deleted and the number of the deleted amino acid residue is 1 to 10, 1 to 5, or 1 to 3, for example, 1 or 2;
(10'-c) in which replacement in the above 10'-a and deletion in the above 10'-b are combined;
(10'-d) in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
(10'-e) in which replacement in the above 10'-a and addition in the above 10'-d are combined;
(10'-f) in which deletion in the above 10'-b and addition in the above 10'-d are combined;
(10'-g) in which replacement in the above 10'-a, deletion in the above 10'-b, and addition in the above 10'-d are combined; and
(10'-h) showing 80% or more, 85% or more, 90% or more, or 95% or more identity, or 98% or more or 99% identity to the amino acid sequence.

(11') a hNAGLU mutant, without adding a mutation to arginine at position 210, glutamic acid at position 36, serine at position 37, and serine at position 45 of the amino acid sequence represented by SEQ ID NO: 31:
(11'-a) in which an amino acid residue constituting the amino acid sequence is replaced with another amino acid residue and the number of the replaced amino acid residue is 1 to 10, 1 to 5, or 1 to 3, for example, 1 or 2;
(11'-b) in which an amino acid residue constituting the amino acid sequence is deleted and the number of the deleted amino acid residues is 1 to 10, 1 to 5, or 1 to 3, for example, 1 or 2;
(11'-c) in which replacement in the above 11'-a and deletion in the above 11'-b are combined;
(11'-d) in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
(11'-e) in which replacement in the above 11'-a and addition in the above 11'-d are combined;
(11'-f) in which deletion in the above 11'-b and addition in the above 11'-d are combined;
(11'-g) in which replacement in the above 11'-a, deletion in the above 11'-b, and addition in the above 11'-d are combined; and
(11'-h) showing 80% or more, 85% or more, 90% or more, or 95% or more identity, or 98% or more or 99% identity to the amino acid sequence.

(12') a hNAGLU mutant, without adding a mutation to arginine at position 209, isoleucine at position 54, and lysine at position 620 of the amino acid sequence represented by SEQ ID NO: 33:
(12'-a) in which an amino acid residue constituting the amino acid sequence is replaced with another amino acid residue and the number of the replaced amino acid residue is 1 to 10, 1 to 5, or 1 to 3, for example, 1 or 2;
(12'-b) in which an amino acid residue constituting the amino acid sequence is deleted and the number of the deleted amino acid residue is 1 to 10, 1 to 5, or 1 to 3, for example, 1 or 2;
(12'-c) in which replacement in the above 12'-a and deletion in the above 12'-b are combined;
(12'-d) in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
(12'-e) in which replacement in the above 12'-a and addition in the above 12'-d are combined;
(12'-f) in which deletion in the above 12'-b and addition in the above 12'-d are combined;
(12'-g) in which replacement in the above 12'-a, deletion in the above 12'-b, and addition in the above 12'-d are combined; and
(12'-h) showing 80% or more, 85% or more, 90% or more, or 95% or more identity, or 98% or more or 99% identity to the amino acid sequence.

(13') a hNAGLU mutant, without adding a mutation to arginine at position 210, isoleucine at position 55, and serine at position 45 of the amino acid sequence represented by SEQ ID NO: 35:
(13'-a) in which an amino acid residue constituting the amino acid sequence is replaced with another amino acid residue and the number of the replaced amino acid residue is 1 to 10, 1 to 5, or 1 to 3, for example, 1 or 2;
(13'-b) in which an amino acid residue constituting the amino acid sequence is deleted and the number of the deleted amino acid residue is 1 to 10, 1 to 5, or 1 to 3, for example, 1 or 2;
(13'-c) in which replacement in the above 13'-a and deletion in the above 13'-b are combined;
(13'-d) in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
(13'-e) in which replacement in the above 13'-a and addition in the above 13'-d are combined;
(13'-f) in which deletion in the above 13'-b and addition in the above 13'-d are combined;
(13'-g) in which replacement in the above 13'-a, deletion in the above 13'-b, and addition in the above 13'-d are combined; and
(13'-h) showing 80% or more, 85% or more, 90% or more, or 95% or more identity, or 98% or more or 99% identity to the amino acid sequence.

(14') a hNAGLU mutant, without adding a mutation to arginine at position 210, lysine at position 621, and serine at position 45 of the amino acid sequence represented by SEQ ID NO: 37:
(14'-a) in which an amino acid residue constituting the amino acid sequence is replaced with another amino acid residue and the number of the replaced amino acid residue is 1 to 10, 1 to 5, or 1 to 3, for example, 1 or 2;
(14'-b) in which an amino acid residue constituting the amino acid sequence is deleted and the number of the deleted amino acid residue is 1 to 10, 1 to 5, or 1 to 3, for example, 1 or 2;
(14'-c) in which replacement in the above 14'-a and deletion in the above 14'-b are combined;
(14'-d) in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
(14'-e) in which replacement in the above 14'-a and addition in the above 14'-d are combined;
(14'-f) in which deletion in the above 14'-b and addition in the above 14'-d are combined;
(14'-g) in which replacement in the above 14'-a, deletion in the above 14'-b, and addition in the above 14'-d are combined; and
(14'-h) showing 80% or more, 85% or more, 90% or more, or 95% or more identity, or 98% or more or 99% identity to the amino acid sequence.

(15') a hNAGLU mutant, without adding a mutation to arginine at position 210, isoleucine at position 55, lysine at position 621, and serine at position 45 of the amino acid sequence represented by SEQ ID NO: 39:
(15'-a) in which an amino acid residue constituting the amino acid sequence is replaced with another amino acid residue and the number of the replaced amino acid residue is 1 to 10, 1 to 5, or 1 to 3, for example, 1 or 2;
(15'-b) in which an amino acid residue constituting the amino acid sequence is deleted and the number of the deleted amino acid residue is 1 to 10, 1 to 5, or 1 to 3, for example, 1 or 2;
(15'-c) in which replacement in the above 15'-a and deletion in the above 15'-b are combined;
(15'-d) in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
(15'-e) in which replacement in the above 15'-a and addition in the above 15'-d are combined;
(15'-f) in which deletion in the above 15'-b and addition in the above 15'-d are combined;
(15'-g) in which replacement in the above 15'-a, deletion in the above 15'-b, and addition in the above 15'-d are combined; and
(15'-h) showing 80% or more, 85% or more, 90% or more, or 95% or more identity, or 98% or more or 99% identity to the amino acid sequence.

The hNAGLU mutant in one embodiment of the present invention exhibits high expression level when expressed in host cells as a recombinant protein, compared with wild-type hNAGLU expressed in host cells as a recombinant protein under the same conditions. This type of hNAGLU mutant is referred to in the present specification as a high-expression type hNAGLU mutant. The high-expression type hNAGLU mutant, when produced as a recombinant protein, can increase the production efficiency compared to the wild-type hNAGLU, thus reducing the production cost. Herein, "under the same conditions" refers to that the expression vectors, host cells, culture conditions, and the like are the same.

A gene encoding a hNAGLU mutant whose expression level is high compared to wild-type hNAGLU when expressed as a recombinant protein in host cells can be obtained, for example, by the following method. Genes encoding mutated hNAGLU and wild-type hNAGLU are each incorporated into the same type of expression vector, and the same type of host cell is transformed using each of those to obtain cells in which each expression vector is introduced. These cells are then cultured under the same conditions and hNAGLU obtained in the culture supernatant is quantified. The gene introduced into the cells showing a higher quantification value than the quantification value of the cells in which the gene encoding wild-type hNAGLU is introduced, is identified as the gene encoding the high-expression type hNAGLU mutant of interest. Herein, as the quantification value, in addition to the quantification value of hNAGLU as a protein, the enzyme activity value of hNAGLU can also be used. For example, the total amount of hNAGLU enzyme activity contained in the culture supernatant can also be used as a quantification value.

Introduction of mutations into wild-type hNAGLU can be performed using a method of randomly introducing mutations into the gene encoding wild-type hNAGLU. For example, by incorporating a gene encoding wild-type hNAGLU into a vector, and allowing a mutagen (radiation, mutagenic substance, and the like) to act on a host cell transformed with the vector, mutations can be introduced into the gene encoding wild-type hNAGLU.

Introduction of mutations into wild-type hNAGLU can be performed using a method of introducing a mutation at a predetermined site in the gene encoding wild-type hNAGLU. For example, such gene mutation can be introduced by chemically synthesizing a gene having a mutation at a predetermined site.

A high-expression type hNAGLU mutant can be produced as a recombinant protein by culturing host cells transformed with an expression vector incorporating a gene encoding the mutant.

The host cells used at this time are not particularly limited as long as it is possible to express the high-expression type hNAGLU mutant by introducing such an expression vector, and may be any of eukaryotic cells such as mammalian cells, yeast, plant cells, and insect cells, and prokaryotic cells such as *Escherichia coli* and *Bacillus subtilis,* but mammalian cells are particularly preferable.

When mammalian cells are used as host cells, the types of mammalian cells are not particularly limited, but cells derived from humans, mice, or Chinese hamsters are preferable, CHO cells derived from Chinese hamster ovary cells or NS/0 cells derived from mouse myeloma are particularly preferable. In this case, the expression vector used for incorporating and expressing the DNA fragment encoding the high-expression type hNAGLU mutant can be used without particular limitation as long as the expression of the gene is caused when introduced into mammalian cells. The gene incorporated into the expression vector is placed downstream of a DNA sequence (gene expression control site) capable of adjusting the frequency of transcription of the gene in mammalian cells. Gene expression control sites that can be used in the present invention includes, for example, cytomegalovirus-derived promoter, SV40 early promoter, human elongation factor-1α (EF-1α) promoter, human ubiquitin C promoter, and the like.

An expression vector is known in which glutamine synthetase (GS) is placed as a selection marker downstream of a gene encoding a protein of interest via an internal ribosome entry site (IRES) (International Patent Publication WO2012/063799, WO2013/161958). The expression vectors described in these documents can be particularly preferably used for producing high-expression type hNAGLU mutants.

For example, an expression vector for expressing a protein of interest, which is an expression vector containing a gene encoding the protein at a first gene expression control site and downstream thereof, and a gene encoding glutamine synthetase at an internal ribosome entry site further downstream and still further downstream thereof, and further containing a dihydrofolate reductase gene or a drug resistance gene downstream of the first gene expression control site or downstream of a second gene expression control site separate from the first gene expression control site, can be preferably used for production of high-expression type hNAGLU mutant. In this expression vector, as the first gene expression control site or the second gene expression control site, cytomegalovirus-derived promoter, SV40 early promoter, human elongation factor-1α promoter (hEF-1α promoter), human ubiquitin C promoter are preferably used, and the hEF-1α promoter is particularly preferable.

In addition, as an internal ribosome entry site, one derived from a 5' untranslated region of a genome of a virus selected from the group consisting of picornaviridae viruses, foot-and-mouth disease virus, hepatitis A virus, hepatitis C virus, coronavirus, bovine enteric virus, Cyler's murine encephalomyelitis virus, and Coxsackie B virus, or a gene selected from the group consisting of human immunoglobulin heavy chain binding protein gene, Drosophila Antennapedia gene, and Drosophila Ultravitrax gene, are preferably used, and an internal ribosome entry site derived from a 5' untranslated region of mouse encephalomyocarditis virus genome is particularly preferable. When using an internal ribosome entry site derived from the 5' untranslated region of the mouse encephalomyocarditis virus genome, in addition to the wild-type one, one in which some of the plurality of initiator codons contained in the wild-type internal ribosome entry site are destroyed can also be preferably used. In addition, in this expression vector, the drug resistance gene preferably used is preferably the puromycin or neomycin resistance gene, more preferably the puromycin resistance gene.

Further, for example, an expression vector for expressing a protein of interest, which is an expression vector containing a human elongation factor-1α promoter, a gene encoding the protein downstream thereof, and a gene encoding glutamine synthetase at the internal ribosome entry site derived from the 5' untranslated region of the mouse encephalomyocarditis virus genome, and further downstream, and a dihydrofolate reductase gene at another gene expression control site and downstream thereof, in which the internal ribosome entry site is partially destroyed in the plurality of initiator codons contained in the wild-type internal ribosome entry site, can be preferably used for production of high-expression type hNAGLU mutants. Examples of such expression vectors include the expression vectors described in WO2013/161958.

In addition, for example, an expression vector for expressing a protein of interest, which is an expression vector containing a human elongation factor-1α promoter, a gene encoding the protein downstream thereof, and a gene encoding glutamine synthetase at the internal ribosome entry site derived from the 5' untranslated region of the mouse encephalomyocarditis virus genome further downstream and still further downstream, and further containing a drug resistance gene at another gene expression control site and downstream thereof, in which the internal ribosome entry site is partially destroyed in the plurality of initiator codons contained in the wild-type internal ribosome entry site, can be preferably used for production of high-expression type hNAGLU mutants. Such expression vectors include pE-mIRES-GS-puro described in WO2012/063799 and pE-mIRES-GS-mNeo described in WO2013/161958.

There are three initiator codons (ATG) at the 3' terminus of the internal ribosome entry site derived from the 5' untranslated region of the wild-type mouse encephalomyocarditis virus genome. The above pE-mIRES-GS-puro and pE-mIRES-GS-mNeo are expression vectors having an IRES with partially destroyed initiator codons.

hNAGLU mutants (including high-expression type hNAGLU mutants) can be expressed in cells or media by culturing host cells in which an expression vector incorporating a gene encoding the hNAGLU mutant is introduced. Methods for expressing hNAGLU mutants when mammalian cells are the host cells are detailed below.

The medium for culturing mammalian cells is not particularly limited as long as mammalian cells are to be cultured and proliferated, but serum-free medium is preferably used. In the present invention, as the serum-free medium used as the recombinant protein production medium, for example, a medium containing 3 to 700 mg/L of amino acids, 0.001 to 50 mg/L of vitamins, 0.3 to 10 g/L of monosaccharides, 0.1 to 10000 mg/L of inorganic salts, 0.001 to 0.1 mg/L of trace elements, 0.1 to 50 mg/L of nucleosides, 0.001 to 10 mg/L of fatty acids, 0.01 to 1 mg/L of biotin, 0.1 to 20 µg/L of hydrocortisone, 0.1 to 20 mg/L of insulin, 0.1 to 10 mg/L of vitamin B12, 0.01 to 1 mg/L putrescine, 10 to 500 mg/L of sodium pyruvate, and a water-soluble iron compound, is preferably used. If desired, thymidine, hypoxanthine, conventional pH indicators, antibiotics and the like may be added to the medium.

As a serum-free medium used as medium for recombinant protein production, a DMEM/F12 medium (mixed medium of DMEM and F12) may be used as a basal medium, and each of these media is well known to those skilled in the art. Furthermore, as a serum-free medium, DMEM (HG) HAM modified (R5) medium containing sodium bicarbonate, L-glutamine, D-glucose, insulin, sodium selenite, diaminobutane, hydrocortisone, iron(II) sulfate, asparagine, aspartic acid, serine, and polyvinyl alcohol may be used. Furthermore, commercially available serum-free media such as CD OPTICHO^{™} medium, CHO-S-SFM II medium, or CD CHO medium (Thermo Fisher Scientific Inc., formerly Life Technologies), IS CHO-V^{™} medium (Irvine Scientific), EX-CELL^{™}302 medium, or EX-CELL^{™}325-PF medium (SAFC Biosciences) or the like can also be used as a basal medium.

In the high-expression type hNAGLU mutant, when mammalian cells introduced with an expression vector incorporating a gene encoding this are cultured in the serum-free medium described above to express the recombinant protein, an expression level of at least 1.1 times or more, 1.5 times or more, 2 times or more, 4 times or more, 5 times or more, or 6 times or more, for example, 1.5 to 4 times, 2 to 5 times, and 2 to 8 times is obtained, compared with wild-type hNAGLU expressed as a recombinant protein under the same conditions. The mammalian cells used at this time are CHO cells, NS/0 cells, and the like, and particularly CHO cells.

By culturing host cells encoding hNAGLU mutants, the recombinant hNAGLU mutants expressed intracellularly or in the medium can be separated from impurities and purified by methods such as column chromatography. Purified hNAGLU mutants can be used as pharmaceuticals. In particular, the hNAGLU mutant can be used as a medicament for treating mucopolysaccharidosis type IIIB (MPS-IIIB), known as Sanfilippo syndrome type B.

A medicament containing a hNAGLU mutant as an active ingredient can be administered intravenously, intramuscularly, intraperitoneally, subcutaneously, or intracerebroventricularly as an injection. These injections can be supplied as freeze-dried formulations or aqueous solutions. When an aqueous solution is used, the aqueous solution may fill a vial, or may be supplied as a pre-filled preparation in which a syringe is filled in advance. When a freeze-dried formulation is used, the freeze-dried formulation is dissolved in an aqueous medium and reconstituted before use.

The hNAGLU mutant in one embodiment of the present invention can make conjugate with an antibody. For example, the hNAGLU mutant in one embodiment of the present invention can be a conjugate with an antibody capable of specifically binding to a receptor on cerebrovascular endothelial cells. By forming a conjugate with an antibody capable of specifically binding to a receptor on cerebrovascular endothelial cells, the hNAGLU mutants can cross the blood-brain barrier (BBB) and a function in the central nervous system (CNS) can be exhibited. Examples of the receptor on cerebrovascular endothelial cells include, but are not limited to, an insulin receptor, a transferrin receptor, a leptin receptor, a lipoprotein receptor, and an IGF receptor. In addition, the receptor is preferably a human-derived receptor.

In the present invention, the term "antibody" mainly refers to a human antibody, a mouse antibody, a humanized antibody, an antibody derived from camelids (including alpacas), a chimeric antibody of a human antibody and another mammalian antibody, and a chimeric antibody of a mouse antibody and another mammalian antibody, but is not limited to these as long as it has the property of specifically binding to a specific antigen, and is no particular limitation to the animal species from which the antibody derives.

In the present invention, the term "human antibody" refers to an antibody of which the entire protein is encoded by human-derived genes. However, "human antibody" also includes an antibody encoded by a gene in which a mutation is added to the original human gene without changing the original amino acid sequence for the purpose of increasing the expression efficiency of the gene. In addition, an antibody prepared by combining two or more genes encoding a human antibody and replacing a part of one human antibody with a part of another human antibody is also a "human antibody." Human antibodies have three complementarity determining regions (CDR) of the light chain and three complementarity determining regions (CDR) of the heavy chain. The three CDRs of the light chain are referred to as CDR1, CDR2, and CDR3 in order from the N-terminus side. The three CDRs of the heavy chain are also referred to as CDR1, CDR2, and CDR3 in order from the N-terminus side. A human antibody also includes the antibody in which the antigen specificity, affinity, and the like of the human antibody is modified by replacing the CDRs of the human antibody with the CDRs of another human antibody.

In the present invention, "human antibody" also includes antibody with a mutation such as a replacement, a deletion, and an addition in the amino acid sequence of the original human antibody by modifying the gene of the original human antibody. When an amino acid in the amino acid sequence of the original antibody is replaced with another amino acid, the number of the amino acid to be replaced is preferably 1 to 20, more preferably 1 to 5, and still more preferably 1 to 3. When an amino acid in the amino acid sequence of the original antibody is deleted, the number of the amino acid to be deleted is preferably 1 to 20, more preferably 1 to 5, and still more preferably 1 to 3. In addition, an antibody with mutations that combine these replacement and deletion of the amino acids is also a human antibody. When an amino acid is added, preferably 1 to 20, more preferably 1 to 5, still more preferably 1 to 3 amino acids are added into the amino acid sequence or to the N-terminus or C-terminus of the original antibody. An antibody with mutations that combine these addition, replacement, and deletion of the amino acids is also a human antibody. The amino acid sequence of the mutated antibody preferably exhibits 80% or more identity, more preferably 90% or more identity, and still more preferably 95% or more identity, and even more preferably 98% or more identity to the original antibody amino acid sequence. When adding the above mutation to a human antibody, the mutation can be added to the variable region of the antibody. When the above mutation is added to the variable region of the antibody, the mutation may be added to either the CDR or the framework region of the variable region, but is particularly added to the framework regions. That is, in the present invention, the term "human-derived gene" includes not only the original human-derived gene, but also a gene obtained by modifying the original human-derived gene.

In the present invention, the term "humanized antibody" refers to an antibody in which the amino acid sequence of part of the variable region (for example, all or a part of the CDRs in particular) is derived from a mammal other than human, and the other regions are derived from humans. Examples of a humanized antibody include an antibody prepared by replacing the three complementarity determining regions (CDRs) of the light chain and the three complementarity determining regions (CDRs) of the heavy chain that constitute the human antibody with the CDRs of another mammal. Other mammalian species from which CDRs to be grafted into appropriate positions for human antibodies are derived are not particularly limited as long as the mammalian species are mammals other than human, but are preferably mice, rats, rabbits, horses, or non-human primates, more preferably mice and rats, still more preferably mice. "Humanized antibody" also includes an antibody obtained by introducing a mutation similar to those that can be introduced to the above human antibody in the amino acid sequence of the original humanized antibody.

In the present invention, the term "chimeric antibody" refers to an antibody in which two or more different antibody fragments derived from two or more different species are linked.

A chimeric antibody of a human antibody and another mammalian antibody is an antibody in which a part of the human antibody is replaced by a part of a non-human mammalian antibody. An antibody consists of an Fc region, an Fab region, and a hinge region, which are described below. Specific examples of such a chimeric antibody include a chimeric antibody in which the Fc region is derived from a human antibody while the Fab region is derived from another mammalian antibody. The hinge region is derived from either a human antibody or another mammalian antibody. Conversely, an antibody in which the Fc region is derived from another mammal while the Fab region is derived from a human antibody is included in a chimeric antibody. The hinge region is derived from either a human antibody or another mammalian antibody.

In addition, an antibody can also consist of a variable region and a constant region. Other specific examples of a chimeric antibody include: a chimeric antibody in which the constant region (C_{H}) of the heavy chain and the constant region (C_{L}) of the light chain are derived from a human antibody, while the variable region (V_{H}) of the heavy chain and the variable region (V_{L}) of the light chain is derived from another mammalian antibody; and conversely, a chimeric antibody in which the constant region (C_{H}) of the heavy chain and the constant region (C_{L}) of the light chain are derived from another mammalian antibody, while the variable region (V_{H}) of the heavy chain and the variable region (V_{L}) of the light chain are derived from human antibody. Here, other mammalian species are not particularly limited as long as the mammalian species are mammals other than human, but are preferably mice, rats, rabbits, horses, or non-human primates, such as mice.

Antibodies in one embodiment of the present invention have a basic structure consisting of a total of four polypeptide chains, that is, two immunoglobulin light chains (or simply "light chains") and two immunoglobulin heavy chains (or simply "heavy chains"). However, in the present invention, in addition to the antibody having this basic structure, the term "antibody" also includes:
(1) an antibody consisting of a total of two polypeptide chains, one light chain, and one heavy chain;
(2) an antibody consisting of the Fab region in which the Fc region is deleted from the basic structure of the antibody in the original meaning, and an antibody consisting of the Fab region and all or a part of the hinge region (including Fab, and F(ab') and F(ab')₂);
(3) a single-chain antibody comprising a light chain which is linked, on the C-terminal side thereof, to a linker sequence which in turn is linked, on the C-terminal side thereof, to a heavy chain;
(4) a single-chain antibody comprising a heavy chain which is linked, on the C-terminal side thereof, to a linker sequence which in turn is linked, on the C-terminal side thereof, to a light chain;
(5) an antibody consisting of the Fc region in which the Fab region is deleted from the basic structure of an antibody in the original meaning, and modified to have a property that the amino acid sequence of the Fc region specifically binds to a specific antigen (Fc antibody); and
(6) a single domain antibody, which is described later.

The antibody in one embodiment of the present invention is an antibody derived from camelids (including alpacas). Some camelid antibodies consist of two heavy chains linked by disulfide bonds. An antibody consisting of these two heavy chains is referred to as a heavy chain antibody. VHH is an antibody consisting of one heavy chain containing a heavy chain variable region that constitutes a heavy chain antibody, or an antibody consisting of one heavy chain lacking a constant region (CH) that constitutes a heavy chain antibody. VHH is also one of the antibodies in the embodiments of the present invention. In addition, an antibody consisting of two light chains linked by disulfide bonds is also one of the antibodies in the embodiments of the present invention. An antibody consisting of these two light chains is referred to as a light chain antibody. An antibody obtained by adding a mutation to the amino acid sequence of a camelid antibody in order to reduce antigenicity occurring when the antibody derived from camelid (including VHH) is administered to humans is also an antibody in one embodiment of the present invention. When a mutation is added to amino acid of a camelid antibody, the same mutation as to be added to the antibody described in the present specification can be added.

The antibody in one embodiment of the present invention is a shark-derived antibody. Shark antibodies consist of two heavy chains linked by disulfide bonds. An antibody consisting of these two heavy chains is referred to as a heavy chain antibody. VNAR is an antibody consisting of one heavy chain containing a heavy chain variable region that constitutes a heavy chain antibody, or an antibody consisting of one heavy chain lacking a constant region (CH) that constitutes a heavy chain antibody. VNAR is also one of the antibodies in the embodiments of the present invention. An antibody obtained by adding a mutation to the amino acid sequence of the shark antibodies in order to reduce antigenicity when the shark-derived antibody (including VNAR) is administered to humans is also an antibody in one embodiment of the present invention. When mutations are added to amino acid of the shark antibodies, the same mutations as those that can be added to the antibodies described in the present specification can be added. An antibody obtained by humanizing shark antibody is also one of the antibodies in the embodiments of the present invention.

An antibody having a basic structure consisting of a total of four polypeptide chains, two light chains and two heavy chains, has three complementarity determining regions (CDR) in the variable region (V_{L}) of the light chain, and three complementarity determining regions (CDR) in the heavy chain variable region (V_{H}). The three CDRs of the light chain are referred to as CDR1, CDR2, and CDR3 in order from the N-terminus side. The three CDRs of the heavy chain are also referred to as CDR1, CDR2, and CDR3 in order from the N-terminus side. However, even when all or a part of these CDRs are incomplete or absent, the antibody having a basic structure is included in antibodies as long as it has the property of specifically binding to a specific antigen. Regions other than the CDRs of variable regions of the light chain and the heavy chain (V_{L} and V_{H}) are referred to as framework regions (FR). FRs are referred to as FR1, FR2, FR3, and FR4 in order from the N-terminus side. Normally, CDRs and FRs are present in order of FR1, CDR1, FR2, CDR2, FR3, CDR3, and FR4 from the N-terminus side.

In one embodiment of the present invention, the antibody also includes the antibody with a mutation such as replacement, deletion, and addition in the amino acid sequence of the original antibody. When an amino acid in the amino acid sequence of the original antibody is replaced with another amino acid, the number of the amino acid to be replaced is preferably 1 to 20, more preferably 1 to 5, and still more preferably 1 to 3. When an amino acid in the amino acid sequence of the original antibody is deleted, the number of the amino acid to be deleted is preferably 1 to 20, more preferably 1 to 5, and still more preferably 1 to 3. In addition, an antibody with mutations that combine these replacement and deletion of amino acids is also an antibody. When an amino acid is added, preferably 1 to 20, more preferably 1 to 5, still more preferably 1 to 3 amino acids are added into the amino acid sequence or to the N-terminus or C-terminus of the original antibody. An antibody with mutations that combine these addition, replacement, and deletion of amino acids is also an antibody. The amino acid sequence of the mutated antibody preferably exhibits 80% or more identity, more preferably 85% or more identity, and still more preferably 90% or more identity, and 95% or more or 98% or more identity to the original antibody amino acid sequence.

In one embodiment of the present invention, the antibody also includes the antibody with a mutation such as replacement, deletion, and addition in the amino acid sequence in the variable region of the original antibody. When an amino acid in the amino acid sequence of the original antibody is replaced with another amino acid, the number of the amino acid to be replaced is preferably 1 to 10, more preferably 1 to 5, and still more preferably 1 to 3. When an amino acid in the amino acid sequence of the original antibody is deleted, the number of the amino acid to be deleted is preferably 1 to 10, more preferably 1 to 5, and still more preferably 1 to 3. In addition, an antibody with mutations that combine these replacement and deletion of amino acids is also an antibody. When an amino acid is added, preferably 1 to 10, more preferably 1 to 5, still more preferably 1 to 3 amino acids are added into the amino acid sequence or to the N-terminus or C-terminus of the original antibody. An antibody with mutations that combine these addition, replacement, and deletion of amino acids are also an antibody. The amino acid sequence of the mutated antibody preferably exhibits 80% or more identity, more preferably 85% or more identity, and still more preferably 90% or more identity, and 95% or more or 98% or more identity to the original antibody amino acid sequence. When an mutation are added to the variable region of the antibody, the mutation may be added to either the CDR or the framework region of the variable region, but is particularly added to the framework region.

In one embodiment of the present invention, the antibody also includes an antibody with a mutation such as replacement, deletion, and addition in the amino acid sequence in the framework region of the variable region of the original antibody. When an amino acid in the amino acid sequence of the original antibody is replaced with another amino acid, the number of the amino acid to be replaced is preferably 1 to 10, more preferably 1 to 5, and still more preferably 1 to 3. When an amino acid in the amino acid sequence of the original antibody is deleted, the number of the amino acid to be deleted is preferably 1 to 10, more preferably 1 to 5, and still more preferably 1 to 3. In addition, an antibody with mutations that combine these replacement and deletion of amino acids is also antibody. When an amino acid is added, preferably 1 to 10, more preferably 1 to 5, still more preferably 1 to 3 amino acids are added into the amino acid sequence or to the N-terminus or C-terminus of the original antibody. An antibody with mutations that combine these addition, replacement, and deletion of amino acids is also an antibody. The amino acid sequence of the mutated antibody preferably exhibits 80% or more identity, more preferably 85% or more identity, and still more preferably 90% or more identity, and 95% or more or 98% or more identity to the original antibody amino acid sequence.

In one embodiment of the present invention, the antibody also includes the antibody with a mutation such as replacement, deletion, and addition in the amino acid sequence in CDR region in the variable region of the original antibody. When an amino acid in the amino acid sequence of the original antibody is replaced with another amino acid, the number of the amino acid to be replaced is preferably 1 to 5, more preferably 1 to 3, and still more preferably 1 or 2. When an amino acid in the amino acid sequence of the original antibody is deleted, the number of the amino acid to be deleted is preferably 1 to 5, more preferably 1 to 3, and still more preferably 1 or 2. In addition, an antibody with mutations that combine these replacement and deletion of amino acids is also an antibody. When an amino acid are added, preferably 1 to 5, more preferably 1 to 3, still more preferably 1 or 2 amino acids are added into the amino acid sequence or to the N-terminus or C-terminus of the original antibody. An antibody with mutations that combine these addition, replacement, and deletion of amino acids is also an antibody. The amino acid sequence of the mutated antibody preferably exhibits 80% or more identity, more preferably 85% or more identity, and still more preferably 90% or more identity, and 95% or more or 98% or more identity to the original antibody amino acid sequence.

The identity between the amino acid sequence of the original antibody and the amino acid sequence of the mutated antibody can be easily calculated using a well-known homology calculation algorithm. Examples of such algorithms include BLAST (Altschul SF. J Mol. Biol. 215. 403-10, (1990)), Pearson and Lipman's similarity search method (Proc. Natl. Acad. Sci. USA. 85. 2444 (1988)), and Smith and Waterman's local homology algorithm (Adv. Appl. Math. 2. 482-9 (1981)).

In one embodiment of the present invention, Fab refers to a molecule in which one light chain containing the variable region and the C_{L} region (constant region of light chain) and one heavy chain containing the variable region and C_{H}1 region (part 1 of constant region of heavy chain) bind to each other by a disulfide bond between cysteine residues present in each of the chains. In Fab, the heavy chain may further contain a part of the hinge region in addition to the variable region and the C_{H}1 region (part 1 of constant region of heavy chain), but the hinge region in this case lacks a cysteine residue that is present in the hinge region and bind the heavy chains of the antibody to each other. In Fab, the light chain and heavy chain bind to each other by a disulfide bond formed between the cysteine residue present in the light chain constant region (C_{L} region) and the cysteine residue present in the heavy chain constant region (C_{H}1 region) or the hinge region. A heavy chain that form the Fab is referred to as a Fab heavy chain. Fab consists of one light chain and one heavy chain since Fab lacks the cysteine residues that are present in the hinge region and bind the heavy chains of the antibody to each other. The light chain that constitutes the Fab contains a variable region and a C_{L} region. A heavy chain that constitutes Fab may consist of a variable region and a C_{H}1 region, or may contain a part of the hinge region while retaining the variable region and C_{H}1 region. However, in this case, the hinge region is selected not to contain cysteine residues binding the heavy chains to each other such that no disulfide bond is formed between the two heavy chains at the hinge region. In F(ab'), the heavy chain contains all or a part of the hinge region containing the cysteine residues binding the heavy chains to each other, in addition to the variable region and C_{H}1 region. F(ab')₂ refers to a molecule in which two F(ab')s bind to each other by disulfide bonds between the cysteine residues that are present in the hinge regions of each other. Heavy chains forming F(ab') or F(ab')₂ are referred to as Fab' heavy chains. In addition, a polymer such as a dimer and a trimer in which a plurality of antibodies bind to each other directly or via linker(s) is also an antibody. Furthermore, not limited to these, any antibody containing a part of an antibody molecule and having the property of specifically binding to an antigen is also included in the "antibody" of the present invention. That is, the term "light chain" in the present invention includes one derived from a light chain and having the amino acid sequence of all or a part of the variable region thereof. In addition, the term "heavy chain" includes those derived from a heavy chain and having the amino acid sequence of all or a part of the variable region thereof. Thus, for example, one from which the Fc region is deleted is also a heavy chain as long as it has the amino acid sequence of all or a part of the variable region.

Here, the Fc or Fc region refers to a region containing a fragment having a C_{H}2 region (part 2 of the heavy chain constant region) and a C_{H}3 region (part 3 of heavy chain constant region) in the antibody molecule.

Furthermore, the antibody in one embodiment of the present invention also includes
(7) scFab, scF(ab'), and scF(ab')₂, which are formed as a single chain antibody, respectively, by binding the light chain and the heavy chain constituting Fab, F(ab'), or F(ab')₂ shown in (2) above via the linker sequence. Here, in scFab, scF(ab'), and scF(ab')₂, the linker sequence may bind to the C-terminus side of the light chain, and further, the heavy chain may bind to the C-terminus side of the linker sequence. In addition, a linker sequence may bind to the C-terminus side of the heavy chain, and further, the light chain may bind to the C-terminus side of the linker sequence. Furthermore, the antibodies of the present invention also include scFv, which is a single chain antibody obtained by binding the variable region of the light chain and the variable region of the heavy chain to each other via a linker sequence. In scFv, the linker sequence may bind to the C-terminus side of the variable region of the light chain, and further, the variable region of the heavy chain may bind to the C-terminus side of the linker sequence. In addition, the linker sequence may bind to the C-terminus side of the variable region of the heavy chain, and further, the variable region of the light chain may bind to the C-terminus side of the linker sequence.

Furthermore, in addition to full-length antibodies shown in (1) to (7) above, the "antibody" referred in the present specification also includes any antigen-binding fragment (antibody fragment) in which a part of the full-length antibody is deficient and has a broader concept as including (1) to (7). An antigen-binding fragment also includes a heavy chain antibody, a light chain antibody, a VHH a VNAR, and anything lacking in some of these.

The term "antigen-binding fragment" refers to a fragment of an antibody that retains at least a part of specific antigen-binding activity. Examples of the binding fragment include Fab, Fab', F(ab')₂, variable region (Fv), a single chain antibody (scFv) in which a heavy chain variable region (V_{H}) and a light chain variable region (V_{L}) are linked with an appropriate linker, a diabody which is a dimer of polypeptides containing the heavy chain variable region (V_{H}) and the light chain variable region (V_{L}), a minibody which is a dimer of the scFv heavy chain (H chain) bound to a part of the constant region (C_{H}3), and other low-molecular-weight antibodies. However, the antigen-binding fragment is not limited to these molecules as long as it has the ability to bind to antigens.

In one embodiment of the present invention, the term "single chain antibody" refers to a protein that can specifically bind to a specific antigen, in which the linker sequence binds to the C-terminus side of the amino acid sequence containing all or a part of the variable region of the light chain, and further, the amino acid sequence containing all or a part of the variable region of the heavy chain binds to the C-terminus side of the linker sequence. In addition, a protein that can specifically bind to a specific antigen, in which the linker sequence binds to the C-terminus side of the amino acid sequence containing all or a part of the variable region of the heavy chain, and further, the amino acid sequence containing all or a part of the variable region of the light chain binds to the C-terminus side of the linker sequence, is also the "single chain antibody" in the present invention. In the single chain antibody in which the light chain binds to the C-terminus side of the heavy chain via the linker sequence, the Fc region is usually deleted in the heavy chain. The variable region of the light chain has three complementarity determining regions (CDR) that are involved in the antigen specificity of the antibody. Similarly, the variable region of the heavy chain also has three CDRs. These CDRs are the main regions that determine the antigen specificity of the antibody. Thus, a single chain antibody preferably contains all three CDRs of the heavy chain and all three CDRs of the light chain. However, as long as the antigen-specific affinity of the antibody is maintained, a single chain antibody with one or more CDRs deleted can be employed.

In the single chain antibody, the linker sequence placed between the light chain and the heavy chain of the antibody is a peptide chain consisting of preferably 2 to 50, more preferably 8 to 50, still more preferably 10 to 30, even more preferably 12 to 18 or 15 to 25 amino acid residues, for example, 15 or 25 amino acid residues. Such a linker sequence is not limited to the amino acid sequence as long as the anti-hTfR antibody in which both chains are linked by the linker sequence retains the affinity to hTfR, but preferably consists of glycine alone or glycine and serine. For example, the linker sequence includes the amino acid sequence Gly-Ser, the amino acid sequence Gly-Gly-Ser, the amino acid sequence Gly-Gly-Gly, the amino acid sequence Gly-Gly-Gly-Gly-Ser (SEQ ID NO: 3), the amino acid sequence Gly-Gly-Gly-Gly-Gly-Ser (SEQ ID NO: 4), the amino acid sequence Ser-Gly-Gly-Gly-Gly (SEQ ID NO: 5), or the amino acid sequence including a sequence wherein these amino acid sequences are repeated 2 to 10 times, or 2 to 5 times. For example, when the variable region of the light chain binds to the C-terminus side of the amino acid sequence consisting of the entire variable region of the heavy chain via a linker sequence, a linker sequence containing a total of 15 amino acids corresponding to a sequence in which three amino acid sequences Gly-Gly-Gly-Gly-Ser (SEQ ID NO: 3) is consecutively linked is preferable.

In one embodiment of the present invention, a single domain antibody refers to an antibody that has the property of specifically binding to an antigen in a single variable region. Single domain antibodies include antibodies of which variable regions consist only of variable regions of the heavy chain (heavy chain single domain antibodies), and antibodies of which variable regions consist only of variable regions of the light chain (light chain single domain antibodies). VHH and VNAR are types of single domain antibodies.

In one embodiment of the present invention, the term "human transferrin receptor" refers to the membrane protein having the amino acid sequence represented by SEQ ID NO: 57. In one embodiment, the antibody of the present invention specifically binds to the part (extracellular domain of transferrin receptor) from the 89th cysteine residue from the N-terminus side to the C-terminus phenylalanine in the amino acid sequence represented by SEQ ID NO: 57, but is not limited thereto.

As a method for producing an antibody against a desired protein, a method for preparing a recombinant protein using cells in which an expression vector incorporating the gene encoding the protein is introduced, and immunizing an animal such as a mouse using this recombinant protein is generally employed. By extracting antibody-producing cells against the recombinant protein from the immunized animal and fusing the antibody-producing cells with myeloma cells, hybridoma cells capable of producing antibodies against the recombinant protein can be prepared.

In addition, cells that produce antibodies against the protein can also be acquired by immunizing immune system cells obtained from animals such as mice with the desired protein by an in vitro immunization method. When using the in vitro immunization method, the animal species from which the immune system cells are derived is not particularly limited, but is preferably primates including mice, rats, rabbits, guinea pigs, dogs, cats, horses, and humans, more preferably mice, rats, and humans, and still more preferably mice and humans. As mouse immune system cells, for example, splenocytes prepared from mouse spleen can be used. As human immune system cells, cells prepared from human peripheral blood, bone marrow, spleen, and the like can be used. When human immune system cells are immunized by the in vitro immunization method, human antibodies against the recombinant protein can be obtained.

Hybridoma cells capable of producing antibodies can be prepared by immunizing immune system cells by the in vitro immunization method and then fusing the cells with myeloma cells. In addition, mRNA is extracted from cells after immunization to synthesize cDNA, and using this cDNA as a template, a PCR reaction is performed to amplify a DNA fragment containing the genes encoding the immunoglobulin light and heavy chains, and these can be used to artificially reconstruct the antibody gene.

Hybridoma cells as obtained by the above method also include cells that produce antibodies that recognize unintended proteins as antigens. In addition, not all hybridoma cells that produce antibodies to a desired protein produce antibodies that exhibit desired properties such as high affinity to the protein.

Similarly, artificially reconstructed antibody genes include genes encoding antibodies that recognize unintended proteins as antigens. Moreover, not all genes encoding antibodies against a desired protein encode antibodies showing desired properties such as high affinity to the protein.

Therefore, it is necessary to select hybridoma cells that produce antibodies with desired properties from the hybridoma cells as obtained above. Moreover, in the case of artificially reconstructed antibody genes, it is necessary to select a gene encoding an antibody having desired properties from the antibody genes. For example, the methods described in detail below are effective as a method for selecting hybridoma cells that produce antibodies with high affinity (high-affinity antibodies) to a desired protein, or for selecting genes that encode high-affinity antibodies.

For example, when selecting hybridoma cells that produce an antibody with high affinity to a desired protein, a method is used in which, after the protein is added to a plate and retained on the plate, the hybridoma cell culture supernatant is added, and then the antibodies that do not bind to the protein are removed from the plate to measure the amount of antibody retained on the plate. According to this method, the higher the affinity of the antibody contained in the culture supernatant of hybridoma cells added to the plate to the protein, the greater the amount of antibody retained on the plate. Therefore, the amount of antibody retained on the plate can be measured, and the hybridoma cells corresponding to the plate on which more antibodies are retained can be selected as cell lines that produce antibodies with relatively high affinity to the protein. From the cell line selected in this manner, mRNA is extracted to synthesize cDNA, and using this cDNA as a template, a DNA fragment containing a gene encoding an antibody against the protein is amplified by the PCR method, and accordingly, genes encoding high-affinity antibodies can also be isolated.

When selecting a gene encoding an antibody against a protein of interest with high affinity from the above-described artificially reconstructed antibody genes, the artificially reconstructed antibody gene is once incorporated into an expression vector, and this expression vector is introduced into a host cell. At this time, the cells used as host cells are not particularly limited, regardless of prokaryotic cells or eukaryotic cells, as long as the cells can express antibody genes by introducing an expression vector incorporating an artificially reconstructed antibody gene. Cells derived from mammals such as humans, mice, and Chinese hamsters are preferable, and CHO cells derived from Chinese hamster ovaries and NS/0 cells derived from mouse myeloma are particularly preferable. In addition, the expression vector used to incorporate and express the gene encoding the antibody gene can be used without particular limitation as long as the expression vector expresses the gene when introduced into mammalian cells. The gene incorporated into the expression vector is placed downstream of a DNA sequence (gene expression control site) capable of adjusting the frequency of transcription of the gene in mammalian cells. Gene expression control sites that can be used in the present invention includes, for example, cytomegalovirus-derived promoter, SV40 early promoter, human elongation factor-1α (EF-1α) promoter, human ubiquitin C promoter, and the like.

Mammalian cells in which such an expression vector is introduced will express the above-described artificially reconstructed antibody incorporated into the expression vector. When selecting cells that produce antibodies with high affinity against an antibody against a desired protein from the cells that express the artificially reconstructed antibodies obtained in this manner, a method is used in which, after the protein is added to the plate and retained, the culture supernatant of the cells are brought into contact with the protein, then the antibody that does not bind to the protein is removed from the plate, and the amount of antibody retained on the plate is measured. According to this method, the higher the affinity of the antibody contained in the culture supernatant of cells to the protein, the greater the amount of antibody retained on the plate. Therefore, the amount of antibody retained on the plate is measured, the cells corresponding to the plate on which more antibodies are retained can be selected as cell lines that produce antibodies with relatively high affinity to the protein, and further, a gene encoding an antibody with a high affinity to the protein can be selected. From a cell line selected in this manner, a gene encoding a high-affinity antibody can also be isolated by amplifying a DNA fragment containing a gene encoding an antibody against the protein using the PCR method.

Methods for producing a conjugate of a hNAGLU mutant and an antibody of the present invention include a method to make a conjugation via a non-peptide linker or a peptide linker. As a non-peptide linker, polyethylene glycol, polypropylene glycol, copolymer of ethylene glycol and propylene glycol, polyoxyethylated polyol, polyvinyl alcohol, polysaccharide, dextran, polyvinyl ether, biodegradable polymer, lipid polymer, chitin, hyaluronic acid, a derivative thereof, or a combination thereof can be used. The peptide linker is a peptide chain or a derivative thereof consisting of 1 to 50 amino acids linked by peptide bonds, and covalently binds on each of the N-terminus and the C-terminus to each of the hNAGLU mutant and the antibody to conjugate the hNAGLU mutant and the antibody.

The antibody and the hNAGLU mutant can also bind to each other via a linker sequence or directly by a peptide bond, in which the N-terminus or the C-terminus of the hNAGLU mutant binds to the C-terminus side or the N-terminus side of the heavy chain or the light chain of the antibody. A conjugate obtained by binding the antibody and the hNAGLU mutant in this manner can be obtained as a fusion protein on the 3' terminus side or the 5' terminus side of the cDNA encoding the heavy chain or the light chain of the antibody, directly or by sandwiching a DNA fragment encoding a linker sequence, by incorporating the DNA fragment in which the cDNA encoding the hNAGLU mutant is placed in-frame into an expression vector for mammalian cells, and by culturing mammalian cells introduced with this expression vector. In the mammalian cells, when the DNA fragment encoding the hNAGLU mutant binds to the heavy chain, an expression vector for mammalian cells incorporating the cDNA fragment encoding the light chain of the antibody can also be introduced into the same host cell, and when the DNA fragment encoding the hNAGLU mutant binds to the light chain, an expression vector for mammalian cells incorporating the cDNA fragment encoding the heavy chain of the antibody can also be introduced into the same host cell. When the antibody is a single chain antibody, the fusion protein that binds the antibody and the hNAGLU mutant to each other can be obtained, on the 5' terminus side or 3' terminus side of the cDNA encoding the hNAGLU mutant, directly or by sandwiching a DNA fragment encoding a linker sequence, by incorporating the DNA fragment linked with the cDNA encoding the single chain antibody into an expression vector (for mammalian cells, eukaryotic cells such as yeast, or prokaryotic cells such as Escherichia coli), and by expressing the DNA fragment in these cells in which this expression vector is introduced. An antibody-hNAGLU mutant fusion protein can be produced as a recombinant protein by the above technique.

The medium for culturing mammalian cells in which the expression vector is introduced is not particularly limited as long as mammalian cells are cultured and proliferated, but the serum-free medium is preferably used. In the present invention, as the serum-free medium used as the recombinant protein production medium, for example, a medium containing 3 to 700 mg/L of amino acids, 0.001 to 50 mg/L of vitamins, 0.3 to 10 g/L of monosaccharides, 0.1 to 10000 mg/L of inorganic salts, 0.001 to 0.1 mg/L of trace elements, 0.1 to 50 mg/L of nucleosides, 0.001 to 10 mg/L of fatty acids, 0.01 to 1 mg/L of biotin, 0.1 to 20 µg/L of hydrocortisone, 0.1 to 20 mg/L of insulin, 0.1 to 10 mg/L of vitamin B12, 0.01 to 1 mg/L putrescine, 10 to 500 mg/L of sodium pyruvate, and a water-soluble iron compound, is preferably used. If desired, thymidine, hypoxanthine, conventional pH indicators, antibiotics and the like may be added to the medium.

As a serum-free medium used as a medium for the production of antibody-hNAGLU mutant fusion proteins, a DMEM/F12 medium (mixed medium of DMEM and F12) may be used as a basal medium, and each of these media is well known to those skilled in the art. Furthermore, as a serum-free medium, DMEM (HG) HAM modified (R5) medium containing sodium bicarbonate, L-glutamine, D-glucose, insulin, sodium selenite, diaminobutane, hydrocortisone, iron(II) sulfate, asparagine, aspartic acid, serine, and polyvinyl alcohol may be used. Furthermore, commercially available serum-free media such as CD OPTICHO^{™} medium, CHO-S-SFM II medium, or CD CHO medium (Thermo Fisher Scientific Inc., formerly Life Technologies), IS CHO-V ^{™} medium (Irvine Scientific), EX-CELL^{™}302 medium, or EX-CELL^{™}325-PF medium (SAFC Biosciences) or the like can also be used as a basal medium.

Preferable embodiments of a fusion protein of the antibody and the hNAGLU mutant include the following (1) to (7). That is, the embodiments include:
(1) a fusion protein containing a conjugate in which an hNAGLU mutant binds to the C-terminus of the heavy chain of the antibody directly or via a linker, and the light chain of the antibody;
(2) a fusion protein containing a conjugate in which a hNAGLU mutant binds to the N-terminus of the heavy chain of the antibody directly or via a linker, and the light chain of the antibody;
(3) a fusion protein containing a conjugate in which a hNAGLU mutant binds to the C-terminus of the light chain of the antibody directly or via a linker, and the heavy chain of the antibody; and
(4) a fusion protein containing a conjugate in which a hNAGLU mutant binds to the N-terminus of the light chain of the antibody directly or via a linker, and the heavy chain of the antibody.

When the antibody and the hNAGLU mutant bind to each other via the linker sequence, the linker sequence placed between the antibody and the hNAGLU mutant is a peptide chain consisting of preferably 1 to 60 or 1 to 50, more preferably 1 to 17, still more preferably 1 to 10, and even more preferably 1 to 5 amino acids, and the number of amino acids constituting the linker sequence can be appropriately adjusted to 1, 2, 3, 1 to 17, 1 to 10, 10 to 40, 20 to 34, 23 to 31, 25 to 29, 27, and the like. Such a linker sequence is not limited to the amino acid sequence as long as the antibody linked in this manner retains affinity to the receptor on cerebrovascular endothelial cells, and the hNAGLU mutant linked by the linker sequence can exhibit the physiological activity of the hNAGLU mutant under physiological conditions, and preferably consists of glycine and serine. Examples thereof include one containing either one amino acid of glycine or serine, and one containing the amino acid sequence Gly-Ser, the amino acid sequence Ser-Ser, the amino acid sequence Gly-Gly-Ser, the amino acid sequence Gly-Gly-Gly-Gly-Ser (SEQ ID NO: 58), the amino acid sequence Gly-Gly-Gly-Gly-Gly-Ser (SEQ ID NO: 59), the amino acid sequence Ser-Gly-Gly-Gly-Gly (SEQ ID NO: 60), or the sequence which includes 1 to 10 or 2 to 5 of any of those amino acid sequences consecutively linked. Examples thereof include those having a sequence consisting of 1 to 50 amino acids, a sequence consisting of 2 to 17, 2 to 10, 10 to 40, 20 to 34, 23 to 31, 25 to 29, or 27 amino acids, and the like. For example, one containing the amino acid sequence Gly-Ser can be preferably used as a linker sequence. In addition, one containing a total of 27 amino acid sequences consisting of five consecutive amino acid sequences Gly-Gly-Gly-Gly-Ser (SEQ ID NO: 58) following the amino acid sequence Gly-Ser can be preferably used as a linker sequence. Furthermore, one containing a total of 25 amino acid sequences consisting of five consecutive amino acid sequences Gly-Gly-Gly-Gly-Ser (SEQ ID NO: 58) can be preferably used as a linker sequence.

In addition, in the present invention, when one peptide chain contains a plurality of linker sequences, for convenience, each linker sequence is named as a first linker sequence and a second linker sequence from the N-terminus side.

Specific examples of an antibody that binds to the hNAGLU mutant of the present invention include the following anti-human transferrin receptor antibodies (anti-hTfR antibodies). That is, examples thereof include:
(1) an anti-hTfR antibody, in which the light chain of the antibody contains the amino acid sequence represented by SEQ ID NO: 61 and the heavy chain contains the amino acid sequence represented by SEQ ID NO: 62; and
(2) an anti-hTfR antibody which is an Fab antibody, and in which the light chain of the antibody contains the amino acid sequence represented by SEQ ID NO: 61 and the heavy chain contains the amino acid sequence represented by SEQ ID NO: 63.

Here, not being limited to these, a mutation such as replacement, deletion, addition, and the like can be appropriately added to the above amino acid sequences. The anti-hTfR antibodies of (1) and (2) above are humanized anti-hTfR antibodies.

When an amino acid in the amino acid sequence of the light chain of the anti-human transferrin receptor antibody is replaced with another amino acids, the number of the amino acid to be replaced is preferably 1 to 10, more preferably 1 to 5, still more preferably 1 to 3, and even more preferably 1 or 2. When an amino acid in the amino acid sequence of the light chain is deleted, the number of the amino acid to be deleted is preferably 1 to 10, more preferably 1 to 5, still more preferably 1 to 3, and even more preferably 1 or 2. In addition, mutations that combine these replacement, and deletion of amino acids can also be added.

When an amino acid is added to the amino acid sequence of the light chain of the anti-human transferrin receptor antibody described above, preferably 1 to 10, more preferably 1 to 5, still more preferably 1 to 3, and even more preferably 1 or 2 amino acids are added into the amino acid sequence of the light chain or to the N-terminus side or the C-terminus side. Mutations that combine these addition, replacement, and deletion of amino acids can also be added. The amino acid sequence of the mutated light chain preferably exhibits 80% or more identity, more preferably 90% or more identity, and still more preferably 95% or more identity to the amino acid sequence of the original light chain.

When an amino acid in the amino acid sequence of the heavy chain of the anti-human transferrin receptor antibody is replaced with another amino acid, the number of the amino acid to be replaced is preferably 1 to 10, more preferably 1 to 5, still more preferably 1 to 3, and even more preferably 1 or 2. When an amino acid in the amino acid sequence of the heavy chain is deleted, the number of the amino acid to be deleted is preferably 1 to 10, more preferably 1 to 5, still more preferably 1 to 3, and even more preferably 1 or 2. In addition, mutations that combine these replacement, and deletion of amino acids can also be added.

When an amino acid is added to the amino acid sequence of the heavy chain of the anti-human transferrin receptor antibody described above, preferably 1 to 10, more preferably 1 to 5, still more preferably 1 to 3, and even more preferably 1 or 2 amino acids are added into the amino acid sequence of the heavy chain or to the N-terminus side or the C-terminus side. Mutations that combine these addition, replacement, and deletion of amino acids can also be added. The amino acid sequence of the mutated heavy chain preferably exhibits 80% or more identity, more preferably 90% or more identity, and still more preferably 95% or more identity to the amino acid sequence of the original heavy chain.

The hNAGLU mutant of the present invention can cross the blood-brain barrier and exert its function in the brain when linked to an antibody against a receptor on cerebrovascular endothelial cells, and thus can be used for the production of a blood-administered medicament for the treatment of a disease state of the central nervous system caused by a deficiency of NAGLU. In addition, the hNAGLU mutant kinked to the antibody can be used in a therapeutic method including administration into blood (including intravenous injection such as intravenous infusion) of a therapeutically effective amount to an applicable patient for disease states of the central nervous system caused by deficiency of NAGLU. The hNAGLU mutant binding to the blood-administered antibody can reach not only the brain but also other organs expressing NAGLU. The medicament can also be used to prevent the development of the disease state.

The hNAGLU mutant of the present invention can be used as a medicament that should be administered into the blood and exert its efficacy in the central nervous system (CNS) by conjugated to an antibody against a receptor on cerebrovascular endothelial cells. Such a medicament is generally administered to a patient by intravenous injection such as intravenous drip, subcutaneous injection, or intramuscular injection, but the route of administration is not particularly limited.

The hNAGLU mutants in one embodiment of the present invention can be linked to not only an antibody, but also to another protein. There is no particular limitation to such another protein, but the protein is, for example, a human-derived protein. A conjugate of a hNAGLU mutant and another protein can be obtained by the above-described method for producing the conjugate of the hNAGLU mutant and the antibody. A fusion protein of the hNAGLU mutant and the other protein can be obtained as a recombinant protein by the above-described method for producing a fusion protein with an antibody. One embodiment of the present invention includes a gene encoding a fusion protein of a hNAGLU mutant and another protein, an expression vector incorporated into the gene, and a host cell in which the expression vector is introduced.

The hNAGLU mutant in one embodiment of the present invention can be linked to not only an anti-hTfR antibody, but also to a substance capable of binding to human hTfR. Although the substance is not particularly limited this, but the protein is, for example, human transferrin. Human transferrin is not limited to wild type, but may be a partial fragment or a mutant thereof as long as it has affinity to hTfR. Such a conjugate can cross the blood-brain barrier and exert function in the brain. A fusion protein of the hNAGLU mutant and the human transferrin can be obtained as a recombinant protein by the above-described method for producing a fusion protein with an antibody. One embodiment of the present invention includes a gene encoding a fusion protein of a hNAGLU mutant and a human transferrin, an expression vector incorporating the gene, and a host cell in which the expression vector is introduced.

### Examples

The present invention will be described in more detail below with reference to examples, but the present invention is not intended to be limited to the examples.

### [Example 1] Construction of expression vector of hNAGLU mutant

A DNA fragment having the nucleic acid sequence represented by SEQ ID NO: 41 containing the wild-type hNAGLU gene was synthesized. Using this as a template, PCRs shown in Table 1 as No. 1 to 7 and 11 were performed using the MluI-added 5' primer having the nucleic acid sequence represented by SEQ ID NO: 42 as the forward primer and each of the primers shown in Table 1 as the reverse primer. Table 1 shows the sequence number corresponding to the nucleic acid sequence of each of reverse primers.

### [Table 1]

**Table 1 Primer 1 used in PCR**

| PCR reaction No. | Name of reverse primer | SEQ ID NO: (Nucleic acid sequence) |
|---|---|---|
| 1 | K36E/P37S mutation introduction 3' primer | 44 |
| 2 | L44_G45insS mutation introduction 3' primer | 45 |
| 3 | R129Q mutation introduction 3' primer | 46 |
| 4 | Q209R mutation introduction 3' primer | 47 |
| 5 | E228K mutation introduction 3' primer | 48 |
| 6 | T240V mutation introduction 3' primer | 49 |
| 7 | T320P/E321D mutation introduction 3' primer | 50 |
| 11 | H240K mutation introduction 3' primer | 54 |

Next, using the DNA fragment having the nucleic acid sequence represented by SEQ ID NO: 41 as a template, PCRs were performed using the PCR products obtained by PCR No. 1 to 7 and 11 shown in Table 1 as forward primers and His-tag-NotI-added 3' primer having the nucleic acid sequence represented by SEQ ID NO: 43 as reverse primer, resulting in obtaining PCR products containing genes encoding the hNAGLU mutants shown in Table 2. Table 2 shows the sequence number corresponding to the amino acid sequence of each of hNAGLU mutants, the sequence number corresponding to the nucleic acid sequence encoding each of these, and the number of each of hNAGLU mutants (mutant number).

### [Table 2]

**Table 2 PCR product name 1**

| PCR reaction No. | Name of encoded hNAGLU mutant | SEQ ID NO: (amino acid sequence) | SEQ ID NO: (Nucleic acid sequence) | Mutant number |
|---|---|---|---|---|
| 1 | K36E/P37S hNAGLU mutant | 3 | 4 | 1 |
| 2 | L44_G45insS hNAGLU mutant | 5 | 6 | 2 |
| 3 | R129Q hNAGLU mutant | 7 | 8 | 16 |
| 4 | Q209R hNAGLU mutant | 9 | 10 | 3 |
| 5 | E228K hNAGLU mutant | 11 | 12 | 4 |
| 6 | T240V hNAGLU mutant | 13 | 14 | 17 |
| 7 | T320P/E321D hNAGLU mutant | 15 | 16 | 5 |
| 11 | H240K hNAGLU mutant | 23 | 24 | 19 |

Using a DNA fragment containing the wild-type hNAGLU gene and having the nucleic acid sequence represented by SEQ ID NO: 41 as a template, PCR No. 8 to 10 shown in Table 3 were performed using a primer shown in Table 3 as a forward primer and His-tag-NotI-added 3' primer having the nucleic acid sequence represented by SEQ ID NO: 43 as a reverse primer. Table 3 shows the sequence number corresponding to the nucleic acid sequence of each of the forward primers.

### [Table 3]

**Table 3 Primer 2 used in PCR**

| PCR reaction No. | Name of forward primer | SEQ ID NO: (nucleic acid sequence) |
|---|---|---|
| 8 | S505A/I506V mutation introduction 5' primer | 51 |
| 9 | S526N/A528T mutation introduction 5' primer | 52 |
| 10 | D613Q mutation introduction 5' primer | 53 |

Next, using the DNA fragment having the nucleic acid sequence represented by SEQ ID NO: 41 as a template, PCR was performed using MluI-added 5' primer having the nucleic acid sequence represented by SEQ ID NO: 42 as a forward primer and the PCR product obtained by PCR No. 8 to 10 shown in Table 3 as a reverse primer, to obtain a PCR product containing a gene encoding the hNAGLU mutant shown in Table 4. Table 4 shows the sequence number corresponding to the amino acid sequence of each of hNAGLU mutants, the sequence number corresponding to the nucleic acid sequence encoding these, and the number of each of hNAGLU mutants (mutant number).

### [Table 4]

**Table 4 PCR product name 2**

| PCR reaction No. | Name of encoded hNAGLU mutant | SEQ ID NO: (amino acid sequence) | SEQ ID NO: (nucleic acid sequence) | Mutant number |
|---|---|---|---|---|
| 8 | S505A/I506V hNAGLU mutant | 17 | 18 | 6 |
| 9 | S526N/A528T hNAGLU mutant | 19 | 20 | 7 |
| 10 | D613Q hNAGLU mutant | 21 | 22 | 18 |

Next, the PCR products obtained by PCR No. 1 to 11 and the DNA fragment having the nucleic acid sequence represented by SEQ ID NO: 41 encoding wild-type hNAGLU were treated with restriction enzymes MluI and NotI (Takara Bio Inc.), and separated by agarose gel electrophoresis. After EtBr staining, the band containing the DNA fragment of interest was excised under UV irradiation, and DNA was extracted from the gel using the QIAEX II Gel Extraction Kit (QIAGEN). Similarly, pCI-neo vector (Promega Corporation) and pEI-puro vector were also treated with restriction enzymes MluI and NotI, gel-extracted, and purified. Each restriction enzyme-treated vector was mixed with each of restriction enzyme-treated PCR products, and ligation reaction was performed at 16°C for 30 to 60 minutes using Ligation Mix (Takara Bio Inc.). A similar ligation reaction was also performed for a DNA fragment having the nucleic acid sequence represented by SEQ ID NO: 41 containing the wild-type hNAGLU gene.

In addition, the pEI-puro vector was prepared by the following procedure. A pEF/myc/nuc vector (Invitrogen) was digested with restriction enzymes (KpnI and NcoI) to excise a DNA fragment containing the EF-1a promoter and its first intron, and this DNA fragment was blunt-ended with T4 DNA polymerase. Separately, pCI-neo (Invitrogen) was digested with restriction enzymes (BglII and EcoRI) to excise the region containing the CMV enhancer/promoter and a intron, and then blunt-ended with T4 DNA polymerase. Into this, the region containing the EF-1a promoter and its first intron above (after blunt-end treatment) was inserted. The resultant was named pE-neo vector. The pCAGIpuro vector (Miyahara M. et.al., J. Biol. Chem. 275, 613-618 (2000)) was digested with restriction enzymes (NotI and BamHI) to excise a DNA fragment containing an internal ribosome entry site (IRES) derived from murine encephalomyocarditis virus (EMCV), puromycin resistance gene (Puro^{r}), and a polyadenylation signal (polyA) derived from bovine growth hormone (bGH). Separately, the pE-neo vector was digested with restriction enzymes (NotI and BamHI) to remove a region of approximately 2 kbp containing the neomycin resistance gene (Neo^{r}). Into this the DNA fragment containing the IRES, PuroR, and Poly A derived from and bGH was inserted. The resultant was named pEl-puro vector.

Then, each ligation reaction solution was used to transform *Escherichia coli* (ECOS^{™} X Competent E. coli DHS α, Nippon Gene). In order to confirm whether the obtained transformant retains the plasmid DNA of interest, a single colony was cultured overnight in an LB liquid medium (LB Broth, Sigma-Aldrich), and the FastGene Plasmid Mini Kit (Nippon Genetics Co., Ltd.) was used to purify a small amount of plasmid DNA. The purified plasmid DNA was treated with restriction enzymes Mini and NotI and separated by agarose gel electrophoresis to confirm that the insert DNA of interest was inserted. In addition, it was confirmed by Sanger sequence analysis that the modification of interest was introduced into each of hNAGLU genes. Each plasmid confirmed to incorporate the hNAGLU mutant of interest or wild-type hNAGLU was purified by a conventional method.

### [Example 2] Transient expression of hNAGLU mutant

Transient expression of hNAGLU mutant was performed using a plasmid incorporating a gene encoding each of hNAGLU mutants into the purified pCI-neo vector obtained in Example 1. As a control, a plasmid incorporating a gene encoding wild-type NAGLU into a pCI-neo vector was used.

According to the high titer protocol of the ExpiCHO Expression System (Thermo Fisher Scientific Inc.), ExpiCHO cells were transformed with a plasmid incorporating the gene encoding each of hNAGLU mutants or a plasmid incorporating the gene encoding the wild-type hNAGLU. After transformation, the cells were cultured for 8 days to express each of hNAGLU mutants and wild-type hNAGLU in the culture supernatant. After culturing, the culture solution was centrifuged to collect the culture supernatant.

### [Example 3] Confirmation of expression level of hNAGLU mutants by transient expression (SDS page electrophoresis)

10 µL of the culture supernatant obtained in Example 2 was mixed with 8 µL of 2 × Sample Buffer (Bio-Rad Laboratories) and 2 µL of 2-Mercaptoethanol and heat-denatured under reducing conditions by heating at 100°C for 3 minutes. 5 µL of the heat-denatured sample was added to each well of a 5 to 20% polyacrylamide gel placed in 50 mM Tris buffer solution/380 mM glycine buffer solution (pH 8.3) containing 0.1% SDS, and electrophoresis was performed at a constant current of 25 mA. After electrophoresis, the gel was immersed in Oriole fluorescent gel stain (Bio-Rad Laboratories) and shaken at room temperature for 90 minutes. After washing the gel with pure water, protein bands were detected with a lumino image analyzer (Amersham Imager 600RGB, Cytiva).

### [Example 4] Confirmation of expression level of hNAGLU mutants by transient expression (Western blotting method)

Electrophoresis was performed in the same manner as in Example 3, the nitrocellulose membrane and the gel after electrophoresis were sandwiched between blotting papers immersed in 25 mM Tris buffer solution/192 mM glycine buffer solution containing 20% methanol, and the protein was transferred to the nitrocellulose membrane by energizing a current of 1.0 A and 25 V for 10 minutes in a blotting device. After transfer, the nitrocellulose membrane was immersed in PBST containing 5% skim milk and shaken for 1 hour, then immersed in mouse anti-His tag mAb (Medical & Biological Laboratories Co., Ltd.) solution diluted to 0.4 µg/mL and shaken for 1 hour. After washing the membrane with PBST, the membrane was immersed in a solution of Anti-mouse IgG (H+L), HRP Conjugate (Promega Corporation) diluted to 0.4 µg/mL, shaken for 30 minutes, and washed again with PBST. An HRP detection reagent (Bio-Rad Laboratories) was added dropwise to the transfer surface of the membrane and allowed to react for 5 minutes, and bands corresponding to each of the hNAGLU mutants and the wild-type hNAGLU were detected with a lumino image analyzer.

### [Example 5] Confirmation of expression level of hNAGLU mutants by transient expression (enzyme activity measurement)

A sample solution was prepared by diluting the culture supernatant obtained in Example 2 10-fold with 100 mM citrate buffer solution (pH 4.2) containing 0.1% BSA. A standard solution was prepared by serially diluting 4-MU (4-Methylumbelliferone, Sigma-Aldrich) from 400 to 35.12 µM with 100 mM citrate buffer solution (pH 4.2) containing 0.1% BSA. A substrate solution was prepared by diluting 4-Methylumbelliferyl-N-acetyl-α-D-glucosaminide (Sigma-Aldrich), which is an artificial substrate of NAGLU, to 1 mmol/L with 100 mM citrate buffer solution (pH 4.2) containing 0.1% BSA. 25 µL of the sample solution or standard solution was added to each well of the microplate, and 25 µL/well of the substrate solution was further added and mixed by stirring with a plate shaker. After heating the plate at 37°C for 1 hour, 150 µL of 200 mmol/L glycine-NaOH buffer solution (pH 10.7) was added to each well to stop the reaction. The fluorescence intensity of released 4-Methylumbelliferone (4-MU) was measured with a fluorescence plate reader (Gemini XPS, Molecular Devices) (excitation wavelength: 355 nm, fluorescence wavelength: 460 nm). A calibration curve was prepared based on the measurement results of the standard solution, and the measurement value of each sample solutions was interpolated to obtain the enzyme activity level.

### [Example 6] Confirmation of expression level of hNAGLU mutants by transient expression (results)

FIG. 1 shows the measurement results of the expression levels of the hNAGLU mutants by transient expression measured in Examples 2 to 4. Table 5 shows the expression level of each of hNAGLU mutants as a relative value when the expression level of the wild type is set to 1 based on the enzyme activity measurement results shown in FIG. 1 as a bar graph.

### [Table 5]

**Table 5 Expression of each of hNAGLU mutants by transient expression**

| (Relative level when expression level of wild-type hNAGLU is set to 1) | | |
|---|---|---|
| Name of hNAGLU mutant | Mutant number | Expression level |
| Wild-type hNAGLU | - | 1 |
| K36E/P37S hNAGLU mutant | 1 | 2.5 |
| L44_G45insS hNAGLU mutant | 2 | 3.2 |
| R129Q hNAGLU mutant | 16 | 0.5 |
| Q209R hNAGLU mutant | 3 | 4.4 |
| E228K hNAGLU mutant | 4 | 1.5 |
| T240V hNAGLU mutant | 17 | 0.6 |
| T320P/E321D hNAGLU mutant | 5 | 2.0 |
| S505A/I506V hNAGLU mutant | 6 | 1.5 |
| S526N/A528T hNAGLU mutant | 7 | 1.2 |
| D613Q hNAGLU mutant | 18 | 0.6 |
| H240K hNAGLU mutant | 19 | 0.4 |

Seven mutants (K36E/P37S hNAGLU mutant, L44_G45insS hNAGLU mutant, Q209R hNAGLU mutant, E228K hNAGLU mutant, T320P/E321D hNAGLU mutant, S505A/I506V hNAGLU mutant, S526N/A528T hNAGLU mutant) (mutant numbers 1 to 7) exhibited higher transient expression compared to the wild type. In particular, the Q209R hNAGLU mutant (mutant number 3) exhibited a 4.4-fold higher expression level than the wild-type hNAGLU. On the other hand, R129Q hNAGLU mutant (mutant number 16), S526N/A528T hNAGLU mutant (mutant number 17), D613Q hNAGLU mutant (mutant number 18), and H204K hNAGLU mutant (mutant number 19) exhibit lower transient expression compared to the wild type.

In addition, a positive correlation was observed between the enzyme activity level and the expression level of hNAGLU mutants confirmed on the SDS page electrophoresis shown in FIG. 1(a). Furthermore, a positive correlation was observed between the enzyme activity level and the expression level of the hNAGLU mutants confirmed by the Western blotting method shown in FIG. 1(b).

### [Example 7] Preparation of hNAGLU mutant-expressing bulk cells

Each expression plasmid incorporating a gene encoding each of hNAGLU mutants or wild-type hNAGLU obtained in Example 1 was introduced into a serum-free conditioned strain of CHO-K1 cells using a gene introduction device (Super Electroporator NEPA21, Nepa Gene Co., Ltd.), and selective culture was performed in CD OPTICHO^{™} medium (Thermo Fisher Scientific Inc.) containing 10 µg/mL Puromycin (Thermo Fisher Scientific Inc.). The volume of culture solution was gradually expanded while repeating medium exchange every 3 to 4 days, and the cells were collected when the survival rate of the cells during culture exceeded 90%, and these were used as hNAGLU mutant-expressing bulk cells and wild-type hNAGLU-expressing bulk cells.

### [Example 8] Culture of hNAGLU mutant-expressing bulk cells

Each of the hNAGLU mutant-expressing bulk cells and the wild-type hNAGLU-expressing bulk cell obtained in Example 7 were respectively seeded at a cell density of 2 × 10⁵ cells/mL in CD OPTICHO^{™} medium containing 10 µg/mL Puromycin, and static culture was performed at 37°C in the presence of 5% CO₂. Nine days after the start of culture, the culture supernatant was collected by centrifugation.

### [Example 9] Confirmation of expression level of hNAGLU mutants in bulk cells (enzyme activity measurement)

A sample solution was prepared by diluting the culture supernatant obtained in Example 8 10-fold with citrate buffer solution (pH 4.2) containing 0.1% BSA. A substrate solution was prepared by diluting 4-Methylumbelliferyl-N-acetyl-α-D-glucosaminide, which is an artificial substrate of hNAGLU, to 1 mmol/L with citrate buffer solution (pH 4.2). 25 µL of the sample solution or standard solution was added to each well of the microplate, and 25 µL/well of the substrate solution was further added and mixed by stirring with a plate shaker. After heating the plate at 37°C for 1 hour, 150 µL of 200 mmol/L glycine-NaOH buffer solution (pH 10.7) was added to each well to stop the reaction. The fluorescence intensity of released 4-Methylumbelliferone (4-MU) was measured with a fluorescence plate reader (excitation wavelength: 355 nm, fluorescence wavelength: 460 nm). A calibration curve was prepared based on the measurement results of the standard solution, and the measurement value of each sample solutions was interpolated to obtain the enzyme activity level. The enzyme activity level of each of hNAGLU mutants was obtained as the enzyme activity level of hNAGLU expressed from 1 × 10⁶ cells during culture (nmol/h/1 × 10⁶ cells).

### [Example 10] Confirmation of expression level of hNAGLU mutants in bulk cells (results)

FIG. 2 shows the measurement results of hNAGLU mutant expression levels in bulk cells measured in Example 9. Table 6 shows the expression level of each of hNAGLU mutants as a relative value when the expression level of the wild type is set to 1 based on the enzyme activity measurement results shown in FIG. 2 as a bar graph. Herein, the culture of the bulk cells was repeated 4 times, and the enzyme activity was measured for each.

### [Table 6]

**Table 6 Expression level of each of hNAGLU mutants in bulk cells**

| (Relative level when expression level of wild-type hNAGLU is set to 1) | | |
|---|---|---|
| Name of hNAGLU mutant | Mutant number | Expression level average value (average value) |
| Wild-type hNAGLU | - | 1 |
| K36E/P37S hNAGLU mutant | 1 | 1.5 |
| L44_G45insS hNAGLU mutant | 2 | 1.6 |
| R129Q hNAGLU mutant | 16 | 0.9 |
| Q209R hNAGLU mutant | 3 | 2.2 |
| E228K hNAGLU mutant | 4 | 1.1 |
| T240V hNAGLU mutant | 17 | 0.5 |
| T320P/E321D hNAGLU mutant | 5 | 1.4 |
| S505A/I506V hNAGLU mutant | 6 | 1.3 |
| S526N/A528T hNAGLU mutant | 7 | 1.2 |
| D613Q hNAGLU mutant | 18 | 0.9 |
| H240K hNAGLU mutant | 19 | 0.3 |

Similar to the case of transient expression, seven mutants (K36E/P37S hNAGLU mutant, L44_G45insS hNAGLU mutant, Q209R hNAGLU mutant, E228K hNAGLU mutant, T320P/E321D hNAGLU mutant, S505A/I506V hNAGLU mutant, S526N/A528T hNAGLU mutant) (mutant numbers 1 to 7) had a higher expression level in bulk cells compared to the wild type. In particular, the Q209R hNAGLU mutant (mutant number 3) exhibited a 2.2-fold higher expression level than the wild-type hNAGLU. Further, similar to the case of transient expression, R129Q hNAGLU mutant (mutant number 16), S526N/A528T hNAGLU mutant (mutant number 17), D613Q hNAGLU mutant (mutant number 18), and H204K hNAGLU mutant (mutant number 19) had a lower expression level compared to the wild type.

### [Example 11] Mutation introduction into Q209R hNAGLU mutant and construction of expression vector

The experimental results of Examples 1 to 10 revealed that the Q209R hNAGLU mutant showed the highest expression level among the hNAGLU mutants. Therefore, further mutations were introduced into the Q209R hNAGLU mutant in order to obtain a higher expression hNAGLU mutant. Using the plasmid incorporating the gene encoding the Q209R hNAGLU mutant obtained in Example 1 as a template, PCR No. 12 to 15 shown in Table 7 were performed using the MluI-added 5' primer having the nucleic acid sequence represented by SEQ ID NO: 42 as a forward primer and the primer shown in Table 7 as a reverse primer. Table 7 shows the sequence number corresponding to the nucleic acid sequence of each of reverse primers.

### [Table 7]

**Table 7 Primer 3 used in PCR**

| PCR reaction No. | Name of reverse primer | SEQ ID NO: (nucleic acid sequence) |
|---|---|---|
| 12 | K36E/P37S mutation introduction 3' primer | 44 |
| 13 | L44_G45insS mutation introduction 3' primer | 45 |
| 14 | T320P/E321D mutation introduction 3' primer | 50 |
| 15 | V54I mutation introduction 3' primer | 55 |

Further, using the plasmid incorporating the gene encoding the Q209R hNAGLU mutant obtained in Example 1 as a template, PCR No. 16 shown in Table 8 was performed using the primer shown in Table 8 as a forward primer and the His-tag-NotI-added 3' primer having the nucleic acid sequence represented by SEQ ID NO: 43 as a reverse primer. Table 8 shows the sequence number corresponding to the nucleic acid sequence of the forward primer.

### [Table 8]

**Table 8 Primer 4 used in PCR**

| PCR reaction No. | Name of forward primer | SEQ ID NO: (nucleic acid sequence) |
|---|---|---|
| 16 | R620K mutation introduction 5' primer | 56 |

Then, using the plasmid incorporating the gene encoding the Q209R hNAGLU mutant obtained in Example 1 as a template, PCR No. 17 to 26 shown in Table 9 were performed using the forward primer and the reverse primer shown in Table 9.

### [Table 9]

**Table 9 Primer 5 used in PCR**

| PCR reaction No. | Name of forward primer | Name of reverse primer |
|---|---|---|
| 17 | PCR reaction No. 12 product | His-tag-NotI-added 3' primer |
| 18 | PCR reaction No. 13 product | His-tag-NotI-added 3' primer |
| 19 | PCR reaction No. 14 product | His-tag-NotI-added 3' primer |
| 20 | PCR reaction No. 12 product | L44_G45insS mutation introduction 3' primer |
| 21 | PCR reaction No. 15 product | PCR reaction No. 16 product |
| 22 | PCR reaction No. 13 product | V54I mutation introduction 3' primer |
| 23 | PCR reaction No. 13 product | PCR reaction No. 16 product |
| 24 | PCR reaction No. 22 product | PCR reaction No. 16 product |
| 25 | PCR reaction No. 20 product | His-tag-NotI-added 3' primer |
| 26 | PCR reaction No. 22 product | His-tag-NotI-added 3' primer |

By PCR No. 17 to 19, 21, and 23 to 26 above, DNA fragments encoding hNAGLU mutants shown in Table 10, in which 1 to 3 mutations were further introduced into the Q209R hNAGLU mutant, were amplified. Table 10 shows the sequence number corresponding to the amino acid sequence of each of hNAGLU mutants, the sequence number corresponding to the nucleic acid sequence encoding each of these, and the number of each of hNAGLU mutants (mutant number).

### [Table 10]

**Table 10 PCR product name 3**

| PCR reactio n No. | Name of encoded hNAGLU mutant | SEQ ID NO: (amino acid sequenc e) | SEQ ID NO: (nucleic acid sequenc e) | Mutan t numbe r |
|---|---|---|---|---|
| 17 | K36E/P37S/Q209R hNAGLU mutant | 25 | 26 | 8 |
| 18 | L44_G45insS/Q209R hNAGLU mutant | 27 | 28 | 9 |
| 19 | Q209R/T320P/E321D hNAGLU mutant | 29 | 30 | 10 |
| 25 | K36E/P37S/L44_G45insS/Q20 9R hNAGLU mutant | 31 | 32 | 11 |
| 21 | V54I/Q209R/R620K hNAGLU mutant | 33 | 34 | 12 |
| 26 | L44_G45insS/V54I/Q209R hNAGLU mutant | 35 | 36 | 13 |
| 23 | L44_G45insS/Q209R/R620K hNAGLU mutant | 37 | 38 | 14 |
| 24 | L44_G45insS/V54I/Q209R/R6 20K hNAGLU mutant | 39 | 40 | 15 |

Next, each of PCR products obtained by PCR No. 17 to 19, 21, and 23 to 26 was treated with restriction enzymes MluI and NotI (Takara Bio Inc.), and separated by agarose gel electrophoresis. After EtBr staining, the band containing the DNA fragment of interest was excised under UV irradiation, and DNA was extracted from the gel using the QIAEX II Gel Extraction Kit (QIAGEN). Similarly, pCI-neo vector (Promega Corporation) and pEI-puro vector were also treated with restriction enzymes MluI, NotI, gel-extracted, and purified. Each restriction enzyme-treated vector was mixed with each of restriction enzyme-treated PCR products, and ligation reaction was performed at 16°C for 30 to 60 minutes using Ligation Mix (Takara Bio Inc.).

Then, each of ligation reaction solutions was used to transform *Escherichia coli* (ECOS^{™} X Competent E. coli DH5 α, Nippon Gene). In order to confirm whether the obtained transformant retains the plasmid DNA of interest, a single colony was cultured overnight in an LB liquid medium (LB Broth, Sigma-Aldrich), and the FastGene Plasmid Mini Kit (Nippon Genetics Co., Ltd.) was used to purify a small amount of plasmid DNA. The purified plasmid DNA was treated with restriction enzymes MluI and NotI and separated by agarose gel electrophoresis to confirm that the insert DNA of interest was inserted. In addition, it was confirmed by Sanger sequence analysis that the modification of interest was introduced into each of hNAGLU genes. Each plasmid confirmed to incorporate the hNAGLU mutant of interest was purified by a conventional method.

### [Example 12] Transient expression of hNAGLU mutant

Transient expression of the hNAGLU mutant was performed using a plasmid incorporating the hNAGLU mutant into the pCI-neo vector purified in Example 11. As a control, a plasmid incorporating wild-type hNAGLU into a pCI-neo vector was used.

According to the high titer protocol of the ExpiCHO Expression System (Thermo Fisher Scientific Inc.), ExpiCHO cells were transformed with a plasmid incorporating the gene encoding the hNAGLU mutant or a plasmid incorporating the gene encoding the wild-type hNAGLU. After transformation, the cells were cultured for 8 days to express each of hNAGLU mutants and wild-type hNAGLU in the culture supernatant. After culturing, the culture solution was centrifuged to collect the culture supernatant. In addition, as a negative control, the culture supernatant of non-transformed cells was collected in the same manner.

### [Example 13] Confirmation of expression level of hNAGLU mutants by transient expression (SDS page electrophoresis)

10 µL of the culture supernatant obtained in Example 12 was mixed with 8 µL of 2 × Sample Buffer and 2 µL of 2-Mercaptoethanol and heat-denatured under reducing conditions by heating at 100°C for 3 minutes. 5 µL of the heat-denatured sample was applied to each well of a 5 to 20% polyacrylamide gel placed in 50 mM Tris buffer solution/380 mM glycine buffer (pH 8.3) containing 0.1% SDS, and electrophoresis was performed at a constant current of 25 mA. After electrophoresis, the gel was immersed in Oriole fluorescent gel stain (Bio-Rad Laboratories) and shaken at room temperature for 90 minutes. After washing the gel with pure water, protein bands were detected with a lumino image analyzer.

### [Example 14] Confirmation of expression level of hNAGLU mutants by transient expression (Western blotting method)

Electrophoresis was performed in the same manner as in Example 13, the nitrocellulose membrane and the gel after electrophoresis were sandwiched between blotting papers immersed in 25 mM Tris buffer solution/192 mM glycine buffer solution containing 20% methanol, and the protein was transferred to the nitrocellulose membrane by energizing a current of 1.0 A and 25 V for 10 minutes in a blotting device. After transfer, the nitrocellulose membrane was immersed in PBST containing 5% skim milk and shaken for 1 hour, then immersed in mouse anti-His tag mAb solution diluted to 0.4 µg/mL and shaken for 1 hour. After washing the membrane with PBST, the membrane was immersed in a solution of Anti-mouse IgG (H+L), HRP Conjugate diluted to 0.4 µg/mL, shaken for 30 minutes, and washed again with PBST. An HRP detection reagent was dropped onto the transfer surface of the membrane, reacted for 5 minutes, and detection was conducted with a lumino image analyzer.

### [Example 15] Confirmation of expression level of hNAGLU mutants by transient expression (enzyme activity measurement)

A sample solution was prepared by diluting the culture supernatant obtained in Example 12 10-fold with citrate buffer solution (pH 4.2) containing 0.1% BSA. A substrate solution was prepared by diluting 4-Methylumbelliferyl-N-acetyl-α-D-glucosaminide, which is an artificial substrate of hNAGLU, to 1 mmol/L with citrate buffer solution (pH 4.2). 25 µL/well of the sample solution or standard solution was added to the microplate, and 25 µL of the substrate solution was added to each well and mixed by stirring with a plate shaker. After heating the plate at 37°C for 1 hour, 150 µL/well of 200 mmol/L glycine-NaOH buffer solution (pH 10.7) was added to stop the reaction. The fluorescence intensity of released 4-Methylumbelliferone (4-MU) was measured with a fluorescence plate reader (excitation wavelength: 355 nm, fluorescence wavelength: 460 nm). A calibration curve was prepared based on the measurement results of the standard solution, and the measurement value of each sample solutions was interpolated to obtain the enzyme activity level.

### [Example 16] Confirmation of expression level of hNAGLU mutants by transient expression (results)

FIGS. 3 and 4 show the measurement results of the expression levels of the hNAGLU mutants by transient expression measured in Examples 12 to 15. Table 11 shows the expression level of each of hNAGLU mutants as a relative value when the expression level of the wild type is set to 1based on the enzyme activity measurement results shown in FIGS. 3 and 4 as a bar graph.

### [Table 11]

**Table 11 Expression level of each of hNAGLU mutants in transient expression**

| (Relative level when expression level of wild-type NAGLU is set to 1) | | |
|---|---|---|
| Name of hNAGLU mutant | Mutant number | Expression level |
| Wild-type hNAGLU | - | 1 |
| Q209R hNAGLU mutant | 3 | 3.3 |
| K36E/P37S/Q209R hNAGLU mutant | 8 | 3.7 |
| L44_G45insS/Q209R hNAGLU mutant | 9 | 5.4 |
| Q209R/T320P/E321D hNAGLU mutant | 10 | 4.0 |
| K36E/P37S/L44_G45insS/Q209R hNAGLU mutant | 11 | 5.4 |
| V54I/Q209R/R620K hNAGLU mutant | 12 | 5.9 |
| L44_G45insS/V54I/Q209R hNAGLU mutant | 13 | 5.1 |
| L44_G45insS/Q209R/R620K hNAGLU mutant | 14 | 4.2 |
| L44_G45insS/V54I/Q209R/R620K hNAGLU mutant | 15 | 5.6 |

| | | |
|---|---|---|
| (Note) The expression levels of Q209R hNAGLU mutant and K36E/P37S/L44_G45insS/Q209R hNAGLU mutant are average values of the expression values shown in FIGS. 3 and 4. | | |

The K36E/P37S/Q209R hNAGLU mutant, the L44_G45insS/Q209R hNAGLU mutant, the Q209R/T320P/E321D hNAGLU mutant, the K36E/P37S/L44_G45insS/Q209R hNAGLU mutant, the V54I/Q209R/R620K hNAGLU mutant, the L44_G45insS/V54I/Q209R hNAGLU mutant, the L44_G45insS/Q209R/R620K hNAGLU mutant, and the L44_G45insS/V54I/Q209R/R620K hNAGLU mutant (mutants 8 to 15), which were obtained by further adding mutation(s) to the Q209R hNAGLU mutant, exhibited higher transient expression compared to the Q209R hNAGLU mutant. In particular, the V54I/Q209R/R620K hNAGLU mutant exhibited approximately 1.8-fold transient expression compared to the Q209R hNAGLU mutant (5.9-fold that of the wild type). In addition, a positive correlation was observed between the enzyme activity level and the expression level of hNAGLU mutants confirmed on the SDS page electrophoresis shown in FIGS. 3(b) and 4(a). Furthermore, a positive correlation was observed between the enzyme activity level and the expression level of the hNAGLU mutants confirmed by the Western blotting method shown in FIG. 4(b).

### [Example 17]

The above results show that it is possible to increase the amount of production of recombinant hNAGLU 2 to 5 times by produced as recombinant Q209R hNAGLU mutant instead of recombinant wild-type hNAGLU, when producing recombinant hNAGLU. Further, they show that it is possible to further increase the amount of production of recombinant hNAGLU by produced as recombinant K36E/P37S/Q209R hNAGLU mutant, recombinant L44_G45insS/Q209R hNAGLU mutant, recombinant Q209R/T320P/E321D hNAGLU mutant, recombinant K36E/P37S/L44_G45insS/Q209R hNAGLU mutant, recombinant V54I/Q209R/R620K hNAGLU mutant, recombinant L44_G45insS/V54I/Q209R hNAGLU mutant, recombinant L44_G45insS/Q209R/R620K hNAGLU mutant, and recombinant L44_G45insS/V54I/Q209R/R620K hNAGLU mutant, with further mutation(s) added to the Q209R hNAGLU mutant.

### [Example 18] Construction of cells for expression of fusion protein of anti-human transferrin receptor antibody (anti-hTfR antibody) and hNAGLU mutant

A fusion protein of an anti-human transferrin receptor antibody (anti-hTfR antibody) and a hNAGLU mutant can be produced by the method detailed below.
pE-neo vector and pE-hygr vector, which are Expression vectors, are constructed by the method described in the Patent Literature (WO2018/124121). The pE-neo vector and the pE-hygr vector are digested with MluI and NotI, respectively.

A DNA fragment is synthesized, which encode a protein represented by SEQ ID NO: 64, in that protein the L44_G45insS/Q209R hNAGLU mutant (mutant number 9) linked to C-terminus of the Fab heavy chain of the anti-hTfR antibody having the amino acid sequence represented by SEQ ID NO: 63, via a linker sequence consisting of a total of 15 amino acids and consisting of three consecutive amino acid sequences represented by SEQ ID NO: 3. On the 5' side of this DNA fragment, an MluI sequence and a sequence encoding a leader peptide functioning as a secretory signal are placed in the order from the 5' end, and a NotI sequence is placed on the 3' side. This DNA fragment is digested with MluI and NotI and incorporated between MluI and NotI of pE-neo vector to construct pE-neo (HC-mhNAGLU).

In addition, at the C-terminus of the L44_G45insS/Q209R hNAGLU mutant (mutant number 9), via a linker sequence consisting of a total of 15 amino acids consisting of three consecutive amino acid sequences represented by SEQ ID NO: 3, a DNA fragment encoding the protein represented by SEQ ID NO: 65, to which the Fab heavy chain of the anti-hTfR antibody binds, containing the amino acid sequence represented by SEQ ID NO: 63, is synthesized. On the 5' side of this DNA fragment, an MluI sequence and a sequence encoding a leader peptide functioning as a secretory signal are placed in the order from the 5' end, and a NotI sequence is placed on the 3' side. This DNA fragment is digested with MluI and NotI and incorporated between MluI and NotI of the pE-neo vector to construct pE-neo (mhNAGLU-HC).

A DNA fragment encoding a DNA fragment (SEQ ID NO: 66) encoding the light chain of anti-hTfR containing the amino acid sequence represented by SEQ ID NO: 61 is synthesized. This DNA fragment is digested with MluI and NotI and incorporated between MluI and NotI of the pE-hygr vector to construct pE-hygr (LC).

CHO cells (CHO-K1: obtained from the American Type Culture Collection) were transformed into pE-neo(HC-mhNAGLU) and pE-neo(HC-mhNAGLU), or pE-neo(HC-mhNAGLU) and pE-neo(mhNAGLU-HC), respectively, using GenePulser (Bio-Rad Laboratories), by using the following method. Transformation of cells is generally performed in the following method. 5 × 10⁵ CHO-K1 cells are seeded in a 3.5 cm culture dish added with CD OPTICHO^{™} medium (Life Technologies) and cultured overnight at 37°C and 5% CO₂. The medium is exchanged with OPTLMEM^{™} I medium (Life Technologies), and the cells are suspended at a density of 5 × 10⁶ cells/mL. 100 µL of the cell suspension is collected, and 5 µL of pE-neo(HC-mhNAGLU) and pE-neo(HC-mhNAGLU) plasmid DNA solutions diluted to 100 µg/mL in OPTI-MEM^{™} I medium, is added thereto. Electroporation is performed using GenePulser (Bio-Rad Laboratories) to introduce the plasmid into the cells. Cells are cultured overnight at 37°C and 5% CO₂ and then selectively cultured on CD OPTICHO^{™} medium added with 0.5 mg/mL hygromycin and 0.8 mg/mL G418.

Then, by limiting dilution, the cells selected by selective culture on a 96-well plate are seeded such that no more than 1 cell is seeded per well, and cultured for approximately 10 days such that each cell forms a monoclonal colony. The culture supernatant is collected from wells in which a monoclonal colony is formed, the humanized antibody content in the culture supernatant is examined by ELISA method, and a humanized antibody high-expression cell line is selected.

The ELISA method at this time is generally performed by the following method. 100 µL of a goat anti-human IgG polyclonal antibody solution diluted to 4 µg/mL with 0.05M bicarbonate buffer solution (pH 9.6) was added to each well of a 96-well microtiter plate (Nunc), and the antibody was adsorbed to the plate by standing at room temperature for at least 1 hour. Next, after washing each well three times with PBS-T, 200 µL of Starting Block (PBS) Blocking Buffer (Thermo Fisher Scientific Inc.) is added to each well and the plate is allowed to stand at room temperature for 30 minutes. After washing each well three times with PBS-T, 100 µL of culture supernatant or human IgG standard diluted to an appropriate concentration with PBS containing 0.5% BSA and 0.05% Tween20 (PBS-BT) is added to each well, and the plate is allowed to stand at room temperature for at least 1 hour. After washing the plate three times with PBS-T, 100 µL of HRP-labeled anti-human IgG polyclonal antibody solution diluted with PBS-BT is added to each well, and the plate is allowed to stand at room temperature for at least 1 hour. After washing each well three times with PBS-T, 100 µL of 0.4 mg/mL o-phenylenediamine containing phosphate-citrate buffer solution (pH 5.0) to each well is added, and is allowed to stand at room temperature for 8-20 minutes. Next, 100 µL of 1 mol/L sulfuric acid is added to each well to stop the reaction, and the absorbance of each well at 490 nm is measured using a 96-well plate reader. Cells corresponding to wells with high measurement values can be used as high-expression cell lines for the production of fusion proteins.

### [Example 19] Production of fusion protein between anti-hTfR antibody and hNAGLU mutant

A fusion protein of an anti-hTfR antibody and a hNAGLU mutant can be produced by the following method. The high-expression cell line described in Example 18 is diluted with CD OPTICHO^{™} medium to a cell concentration of approximately 2 × 10⁵ /mL, 200 mL of the cell suspension is added to a 1 L Erlenmeyer flask, and culture is performed for 6 to 7 days at 37°C in a humidified environment of 5% CO₂ and 95% air with an agitation speed of approximately 70 rpm. The culture supernatant is collected by centrifugation and filtered through a 0.22 µm filter (Millipore) to collect the culture supernatant. 5 times the column volume of 20 mM Tris buffer solution (pH 8.0) containing 150 mL NaCl were added to the culture supernatant, and is loaded on a Protein A column (column volume: 1 mL, Bio-Rad Laboratories) pre-equilibrated with 3 times the column volume of 20 mM Tris buffer solution (pH 8.0) containing 150 mM NaCl. Next, after washing the column with 5 times the column volume of the same buffer solution, the adsorbed fusion protein is eluted with 4 times the column volume of 50 mM glycine buffer solution (pH 2.8) containing 150 mM NaCl. The pH of the effluent containing this fusion protein is adjusted to pH 7.0 by adding 1 M Tris buffer solution (pH 8.0). The solution obtained in this manner is stored at 4°C or frozen as a purified product of the fusion protein.

### Industrial Applicability

According to the present invention, a hNAGLU mutant that can be administered as an enzyme replacement therapy for the treatment of patients with mucopolysaccharidosis type IIIB, which is a hNAGLU mutant more efficiently produced as a recombinant protein than wild-type hNAGLU can be provided.

### Sequence Listing Free Text

SEQ ID NO: 1: Wild-type hNAGLU amino acid sequence
SEQ ID NO: 2: Nucleic acid sequence of DNA fragment encoding wild-type hNAGLU, synthetic sequence
SEQ ID NO: 3: Amino acid sequence of hNAGLU mutant number 1
SEQ ID NO: 4: Nucleic acid sequence of DNA fragment encoding hNAGLU mutant number 1, synthetic sequence
SEQ ID NO: 5: Amino acid sequence of hNAGLU mutant number 2
SEQ ID NO: 6: Nucleic acid sequence of DNA fragment encoding hNAGLU mutant number 2, synthetic sequence
SEQ ID NO: 7: Amino acid sequence of hNAGLU mutant number 16
SEQ ID NO: 8: Nucleic acid sequence of DNA fragment encoding hNAGLU mutant number 16, synthetic sequence
SEQ ID NO: 9: Amino acid sequence of hNAGLU mutant number 3
SEQ ID NO: 10: Nucleic acid sequence of DNA fragment encoding hNAGLU mutant number 3, synthetic sequence
SEQ ID NO: 11: Amino acid sequence of hNAGLU mutant number 4
SEQ ID NO: 12: Nucleic acid sequence of DNA fragment encoding hNAGLU mutant number 4, synthetic sequence
SEQ ID NO: 13: Amino acid sequence of hNAGLU mutant number 17
SEQ ID NO: 14: Nucleic acid sequence of DNA fragment encoding hNAGLU mutant number 17, synthetic sequence
SEQ ID NO: 15: Amino acid sequence of hNAGLU mutant number 5
SEQ ID NO: 16: Nucleic acid sequence of DNA fragment encoding hNAGLU mutant number 5, synthetic sequence
SEQ ID NO: 17: Amino acid sequence of hNAGLU mutant number 6
SEQ ID NO: 18: Nucleic acid sequence of DNA fragment encoding hNAGLU mutant number 6, synthetic sequence
SEQ ID NO: 19: Amino acid sequence of hNAGLU mutant number 7
SEQ ID NO: 20: Nucleic acid sequence of DNA fragment encoding hNAGLU mutant number 7, synthetic sequence
SEQ ID NO: 21: Amino acid sequence of hNAGLU mutant number 18
SEQ ID NO: 22: Nucleic acid sequence of DNA fragment encoding hNAGLU mutant number 18, synthetic sequence
SEQ ID NO: 23: Amino acid sequence of hNAGLU mutant number 19
SEQ ID NO: 24: Nucleic acid sequence of DNA fragment encoding hNAGLU mutant number 19, synthetic sequence
SEQ ID NO: 25: Amino acid sequence of hNAGLU mutant number 8
SEQ ID NO: 26: Nucleic acid sequence of DNA fragment encoding hNAGLU mutant number 8, synthetic sequence
SEQ ID NO: 27: Amino acid sequence of hNAGLU mutant number 9
SEQ ID NO: 28: Nucleic acid sequence of DNA fragment encoding hNAGLU mutant number 9, synthetic sequence
SEQ ID NO: 29: Amino acid sequence of hNAGLU mutant number 10
SEQ ID NO: 30: Nucleic acid sequence of DNA fragment encoding hNAGLU mutant number 10, synthetic sequence
SEQ ID NO: 31: Amino acid sequence of hNAGLU mutant number 11
SEQ ID NO: 32: Nucleic acid sequence of DNA fragment encoding hNAGLU mutant number 11, synthetic sequence
SEQ ID NO: 33: Amino acid sequence of hNAGLU mutant number 12
SEQ ID NO: 34: Nucleic acid sequence of DNA fragment encoding hNAGLU mutant number 12, synthetic sequence
SEQ ID NO: 35: Amino acid sequence of hNAGLU mutant number 13
SEQ ID NO: 36: Nucleic acid sequence of DNA fragment encoding hNAGLU mutant number 13, synthetic sequence
SEQ ID NO: 37: Amino acid sequence of hNAGLU mutant number 14
SEQ ID NO: 38: Nucleic acid sequence of DNA fragment encoding hNAGLU mutant number 14, synthetic sequence
SEQ ID NO: 39: Amino acid sequence of hNAGLU mutant number 15
SEQ ID NO: 40: Nucleic acid sequence of DNA fragment encoding hNAGLU mutant number 15, synthetic sequence
SEQ ID NO: 41: Nucleic acid sequence containing gene encoding hNAGLU, synthetic sequence
SEQ ID NO: 42: MluI-added 5' primer, synthetic sequence
SEQ ID NO: 43: His-tag-NotI-added 3' primer, synthetic sequence
SEQ ID NO: 44: K36E/P37S mutation-introducing 3' primer, synthetic sequence
SEQ ID NO: 45: L44_G45insS mutation-introducing 3' primer, synthetic sequence
SEQ ID NO: 46: R129Q mutation-introducing 3' primer, synthetic sequence
SEQ ID NO: 47: Q209R mutation-introducing 3' primer, synthetic sequence
SEQ ID NO: 48: E228K mutation-introducing 3' primer, synthetic sequence
SEQ ID NO: 49: T240V mutation-introducing 3' primer, synthetic sequence
SEQ ID NO: 50: T320P/E321D mutation-introducing 3' primer, synthetic sequence
SEQ ID NO: 51: S505A/I506V mutation-introducing 5' primer, synthetic sequence
SEQ ID NO: 52: S526N/A528T mutation-introducing 5' primer, synthetic sequence
SEQ ID NO: 53: D613Q mutation-introducing 5' primer, synthetic sequence
SEQ ID NO: 54: H204K mutation-introducing 3' primer, synthetic sequence
SEQ ID NO: 55: V54I mutation-introducing 3' primer, synthetic sequence
SEQ ID NO: 56: R620K mutation-introducing 5' primer, synthetic sequence
SEQ ID NO: 57: Amino acid sequence of human transferrin receptor
SEQ ID NO: 58: Example 1 of amino acid sequence of linker
SEQ ID NO: 59: Example 2 of amino acid sequence of linker
SEQ ID NO: 60: Example 3 of amino acid sequence of linker
SEQ ID NO: 61: Amino acid sequence of light chain of anti-hTfR antibody
SEQ ID NO: 62: Amino acid sequence of heavy chain of anti-hTfR antibody
SEQ ID NO: 63: Amino acid sequence of Fab heavy chain of anti-hTfR antibody
SEQ ID NO: 64: Amino acid sequence 1 of fusion protein of hNAGLU mutant number 9 and Fab heavy chain of anti-hTfR antibody
SEQ ID NO: 65: Amino acid sequence 2 of fusion protein of hNAGLU mutant number 9 and Fab heavy chain of anti-hTfR antibody
SEQ ID NO: 66: Nucleic acid sequence encoding amino acid sequence of light chain of anti-hTfR antibody, synthetic sequence
SEQ ID NO: 67: Amino acid sequence of signal peptide of wild-type hNAGLU precursor

## Claims

1. A mutant of human α-N-acetylglucosaminidase (hNAGLU) selected from the group consisting of (1) to (7) below:
(1) a mutant containing an amino acid sequence represented by SEQ ID NO: 3 in which lysine at position 36 in a wild-type hNAGLU amino acid sequence represented by SEQ ID NO: 1 is replaced with glutamic acid and proline at position 37 is replaced with serine, respectively;
(2) a mutant containing an amino acid sequence represented by SEQ ID NO: 5 in which serine is added between leucine at position 44 and glycine at position 45 in the wild-type hNAGLU amino acid sequence represented by SEQ ID NO: 1;
(3) a mutant containing an amino acid sequence represented by SEQ ID NO: 9 in which glutamine at position 209 in the wild-type hNAGLU amino acid sequence represented by SEQ ID NO: 1 is replaced with arginine;
(4) a mutant containing an amino acid sequence represented by SEQ ID NO: 11 in which glutamic acid at position 228 in the wild-type hNAGLU amino acid sequence represented by SEQ ID NO: 1 is replaced with lysine;
(5) a mutant containing an amino acid sequence represented by SEQ ID NO: 15 in which threonine at position 320 in the wild-type hNAGLU amino acid sequence represented by SEQ ID NO: 1 is replaced with proline and glutamic acid at position 321 is replaced with aspartic acid, respectively;
(6) a mutant containing an amino acid sequence represented by SEQ ID NO: 17 in which serine at position 505 in the wild-type hNAGLU amino acid sequence represented by SEQ ID NO: 1 is replaced with alanine and isoleucine at position 506 is replaced with valine; and
(7) a mutant containing an amino acid sequence represented by SEQ ID NO: 19 in which serine at position 526 in the wild-type hNAGLU amino acid sequence represented by SEQ ID NO: 1 is replaced with asparagine and alanine at position 528 is replaced with threonine, respectively.

2. A hNAGLU mutant containing a mutation relative to the hNAGLU mutant containing the amino acid sequence represented by SEQ ID NO: 3 according to claim 1 while glutamic acid at position 36 and serine at position 37 of the amino acid sequence retained, selected from the group consisting of (1'-a) to (1'-h) below:
(1'-a) a hNAGLU mutant in which an amino acid residue constituting the amino acid sequence is replaced with another amino acid residue and the number of the replaced amino acid residues is 1 to 10, 1 to 5, or 1 to 3, for example, 1 or 2;
(1'-b) a hNAGLU mutant in which the amino acid residue constituting the amino acid sequence is deleted and the number of the deleted amino acid residue is 1 to 10, 1 to 5, or 1 to 3, for example, 1 or 2;
(1'-c) a hNAGLU mutant having both of the replacement in the above 1'-a and the deletion in the above 1'-b;
(1'-d) a hNAGLU mutant in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
(1'-e) a hNAGLU mutant having both of the replacement in the above 1'-a and the addition in the above 1'-d;
(1'-f) a hNAGLU mutant having both of the deletion in the above 1'-b and the addition in the above 1'-d;
(1'-g) a hNAGLU mutant having both of the replacement in the above 1'-a, the deletion in the above 1'-b, and the addition in the above 1'-d; and
(1'-h) a hNAGLU mutant showing 80% or more, 85% or more, 90% or more, or 95% or more identity, or 98% or more or 99% identity to the amino acid sequence.

3. A hNAGLU mutant containing a mutation relative to the hNAGLU mutant containing the amino acid sequence represented by SEQ ID NO: 5 according to claim 1 while serine at position 45 of the amino acid sequence retained, selected from the group consisting of (2'-a) to (2'-h) below:
(2'-a) a hNAGLU mutant in which an amino acid residue constituting the amino acid sequence is replaced with another amino acid residue and the number of the replaced amino acid residue is 1 to 10, 1 to 5, or 1 to 3, for example, 1 or 2;
(2'-b) a hNAGLU mutant in which an amino acid residue constituting the amino acid sequence is deleted and the number of the deleted amino acid residue is 1 to 10, 1 to 5, or 1 to 3, for example, 1 or 2;
(2'-c) a hNAGLU mutant having both of the replacement in the above 2'-a and the deletion in the above 2'-b;
(2'-d) a hNAGLU mutant in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
(2'-e) a hNAGLU mutant having both of the replacement in the above 2'-a and the addition in the above 2'-d;
(2'-f) a hNAGLU mutant having both of the deletion in the above 2'-b and the addition in the above 2'-d;
(2'-g) a hNAGLU mutant having all of the replacement in the above 2'-a, the deletion in the above 2'-b, and the addition in the above 2'-d; and
(2'-h) a hNAGLU mutant showing 80% or more, 85% or more, 90% or more, or 95% or more identity, or 98% or more or 99% identity to the amino acid sequence.

4. A hNAGLU mutant containing a mutation relative to the hNAGLU mutant containing the amino acid sequence represented by SEQ ID NO: 9 according to claim 1 while arginine at position 209 of the amino acid sequence retained, selected from the group consisting of (3'-a) to (3'-h) below:
(3'-a) a hNAGLU mutant in which an amino acid residue constituting the amino acid sequence is replaced with another amino acid residue and the number of the replaced amino acid residue is 1 to 10, 1 to 5, or 1 to 3, for example, 1 or 2;
(3'-b) a hNAGLU mutant in which an amino acid residue constituting the amino acid sequence is deleted and the number of the deleted amino acid residue is 1 to 10, 1 to 5, or 1 to 3, for example, 1 or 2;
(3'-c) a hNAGLU mutant having both of the replacement in the above 3'-a and the deletion in the above 3'-b;
(3'-d) a hNAGLU mutant in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
(3'-e) a hNAGLU mutant having both of the replacement in the above 3'-a and the addition in the above 3'-d;
(3'-f) a hNAGLU mutant having both of the deletion in the above 3'-b and the addition in the above 3'-d;
(3'-g) a hNAGLU mutant having all of the replacement in the above 3'-a, the deletion in the above 3'-b, and the addition in the above 3'-d; and
(3'-h) a hNAGLU mutant showing 80% or more, 85% or more, 90% or more, or 95% or more identity, or 98% or more or 99% identity to the amino acid sequence.

5. A hNAGLU mutant containing a mutation relative to the hNAGLU mutant containing the amino acid sequence represented by SEQ ID NO: 11 according to claim 1 while lysine at position 228 of the amino acid sequence retained, selected from the group consisting of (4'-a) to (4'-h) below:
(4'-a) a hNAGLU mutant in which an amino acid residue constituting the amino acid sequence is replaced with another amino acid residue and the number of the replaced amino acid residue is 1 to 10, 1 to 5, or 1 to 3, for example, 1 or 2;
(4'-b) a hNAGLU mutant in which an amino acid residue constituting the amino acid sequence is deleted and the number of the deleted amino acid residue is 1 to 10, 1 to 5, or 1 to 3, for example, 1 or 2;
(4'-c) a hNAGLU mutant having both of the replacement in the above 4'-a and the deletion in the above 4'-b;
(4'-d) a hNAGLU mutant in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
(4'-e) a hNAGLU mutant having both of the replacement in the above 4'-a and the addition in the above 4'-d;
(4'-f) a hNAGLU mutant having both of the deletion in the above 4'-b and the addition in the above 4'-d;
(4'-g) a hNAGLU mutant having all of the replacement in the above 4'-a, the deletion in the above 4'-b, and the addition in the above 4'-d; and
(4'-h) a hNAGLU mutant showing 80% or more, 85% or more, 90% or more, or 95% or more identity, or 98% or more or 99% identity to the amino acid sequence.

6. A hNAGLU mutant containing a mutation relative to the hNAGLU mutant containing the amino acid sequence represented by SEQ ID NO: 15 according to claim 1 while proline at position 320 and aspartic acid at position 321 of the amino acid sequence retained, selected from the group consisting of (5'-a) to (5'-h) below:
(5'-a) a hNAGLU mutant in which an amino acid residue constituting the amino acid sequence is replaced with another amino acid residue and the number of the replaced amino acid residue is 1 to 10, 1 to 5, or 1 to 3, for example, 1 or 2;
(5'-b) a hNAGLU mutant in which an amino acid residue constituting the amino acid sequence is deleted and the number of the deleted amino acid residue is 1 to 10, 1 to 5, or 1 to 3, for example, 1 or 2;
(5'-c) a hNAGLU mutant having both of the replacement in the above 5'-a and the deletion in the above 5'-b;
(5'-d) a hNAGLU mutant in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
(5'-e) a hNAGLU mutant having both of the replacement in the above 5'-a and the addition in the above 5'-d;
(5'-f) a hNAGLU mutant having both of the deletion in the above 5'-b and the addition in the above 5'-d;
(5'-g) a hNAGLU mutant having all of the replacement in the above 5'-a, the deletion in the above 5'-b, and the addition in the above 5'-d; and
(5'-h) a hNAGLU mutant showing 80% or more, 85% or more, 90% or more, or 95% or more identity, or 98% or more or 99% identity to the amino acid sequence.

7. A hNAGLU mutant containing a mutant relative to the hNAGLU mutant containing the amino acid sequence represented by SEQ ID NO: 17 according to claim 1 while alanine at position 505 and valine at position 506 of the amino acid sequence retained, selected from the group consisting of (6'-a) to (6'-h) below:
(6'-a) a hNAGLU mutant in which an amino acid residue constituting the amino acid sequence is replaced with another amino acid residue and the number of the replaced amino acid residue is 1 to 10, 1 to 5, or 1 to 3, for example, 1 or 2;
(6'-b) a hNAGLU mutant in which an amino acid residue constituting the amino acid sequence is deleted and the number of the deleted amino acid residue is 1 to 10, 1 to 5, or 1 to 3, for example, 1 or 2;
(6'-c) a hNAGLU mutant having both of the replacement in the above 6'-a and the deletion in the above 6'-b;
(6'-d) a hNAGLU mutant in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
(6'-e) a hNAGLU mutant having both of the replacement in the above 6'-a and the addition in the above 6'-d;
(6'-f) a hNAGLU mutant having both of the deletion in the above 6'-b and the addition in the above 6'-d;
(6'-g) a hNAGLU mutant having all of the replacement in the above 6'-a, the deletion in the above 6'-b, and the addition in the above 6'-d; and
(6'-h) a hNAGLU mutant showing 80% or more, 85% or more, 90% or more, or 95% or more identity, or 98% or more or 99% identity to the amino acid sequence.

8. A hNAGLU mutant containing a mutation relative to the hNAGLU mutant containing the amino acid sequence represented by SEQ ID NO: 19 according to claim 1 while asparagine at position 526 and threonine at position 528 of the amino acid sequence retained, selected from the group consisting of (7'-a) to (7'-h) below:
(7'-a) a hNAGLU mutant in which an amino acid residue constituting the amino acid sequence is replaced with another amino acid residue and the number of the replaced amino acid residue is 1 to 10, 1 to 5, or 1 to 3, for example, 1 or 2;
(7'-b) a hNAGLU mutant in which an amino acid residue constituting the amino acid sequence is deleted and the number of the deleted amino acid residue is 1 to 10, 1 to 5, or 1 to 3, for example, 1 or 2;
(7'-c) a hNAGLU mutant having both of the replacement in the above 7'-a and the deletion in the above 7'-b;
(7'-d) a hNAGLU mutant in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
(7'-e) a hNAGLU mutant having both of the replacement in the above 7'-a and the addition in the above 7'-d;
(7'-f) a hNAGLU mutant having both of the deletion in the above 7'-b and the addition in the above 7'-d;
(7'-g) a hNAGLU mutant having all of the replacement in the above 7'-a, the deletion in the above 7'-b, and the addition in the above 7'-d; and
(7'-h) a hNAGLU mutant showing 80% or more, 85% or more, 90% or more, or 95% or more identity, or 98% or more or 99% identity to the amino acid sequence.

9. A hNAGLU mutant containing a mutation relative to the hNAGLU mutant containing the amino acid sequence represented by SEQ ID NO: 9 according to claim 4, selected from the group consisting of (8) to (15) below:
(8) a hNAGLU mutant containing the amino acid sequence represented by SEQ ID NO: 25 in which lysine at position 36 is replaced with glutamic acid and proline at position 37 is replaced with serine, respectively;
(9) a hNAGLU mutant containing an amino acid sequence represented by SEQ ID NO: 27 in which serine is added between leucine at position 44 and glycine at position 45;
(10) a hNAGLU mutant containing an amino acid sequence represented by SEQ ID NO: 29 in which threonine at position 320 is replaced with proline and glutamic acid at position 321 is replaced with aspartic acid;
(11) a hNAGLU mutant containing an amino acid sequence represented by SEQ ID NO: 31 in which lysine at position 36 is replaced with glutamic acid, and proline at position 37 is replaced with serine, respectively, and serine is added between leucine at position 44 and glycine at position 45;
(12) a hNAGLU mutant containing an amino acid sequence represented by SEQ ID NO: 33 in which valine at position 54 is replaced with isoleucine and arginine at position 620 is replaced with lysine, respectively;
(13) a hNAGLU mutant containing an amino acid sequence represented by SEQ ID NO: 35 in which valine at position 54 is replaced with isoleucine and serine is added between leucine at position 44 and glycine at position 45;
(14) a hNAGLU mutant containing an amino acid sequence represented by SEQ ID NO: 37 in which arginine at position 620 is replaced with lysine and serine is added between leucine at position 44 and glycine at position 45; and
(15) a hNAGLU mutant containing an amino acid sequence represented by SEQ ID NO: 39 in which valine at position 54 is replaced with isoleucine, and arginine at position 620 is replaced with lysine, respectively, and serine is added between leucine at position 44 and glycine at position 45.

10. A hNAGLU mutant containing a mutation relative to the hNAGLU mutant containing the amino acid sequence represented by SEQ ID NO: 25 according to claim 9 while arginine at position 209, glutamic acid at position 36, and serine at position 37 of the amino acid sequence retained, selected from the group consisting of (8'-a) to (8'-h) below:
(8'-a) a hNAGLU mutant in which an amino acid residue constituting the amino acid sequence is replaced with another amino acid residue and the number of the replaced amino acid residue is 1 to 10, 1 to 5, or 1 to 3, for example, 1 or 2;
(8'-b) a hNAGLU mutant in which an amino acid residue constituting the amino acid sequence is deleted and the number of the deleted amino acid residue is 1 to 10, 1 to 5, or 1 to 3, for example, 1 or 2;
(8'-c) a hNAGLU mutant having both of the replacement in the above 8'-a and the deletion in the above 8'-b;
(8'-d) a hNAGLU mutant in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
(8'-e) a hNAGLU mutant having both of the replacement in the above 8'-a and the addition in the above 8'-d;
(8'-f) a hNAGLU mutant having both of the deletion in the above 8'-b and the addition in the above 8'-d;
(8'-g) a hNAGLU mutant having all of the replacement in the above 8'-a, the deletion in the above 8'-b, and the addition in the above 8'-d; and
(8'-h) a hNAGLU mutant showing 80% or more, 85% or more, 90% or more, or 95% or more identity, or 98% or more or 99% identity to the amino acid sequence.

11. A hNAGLU mutant containing a mutation relative to the hNAGLU mutant containing the amino acid sequence represented by SEQ ID NO: 27 according to claim 9 while arginine at position 210 and serine at position 45 of the amino acid sequence retained, selected from the group consisting of (9'-a) to (9'-h) below:
(9'-a) a hNAGLU mutant in which an amino acid residue constituting the amino acid sequence is replaced with another amino acid residue and the number of the replaced amino acid residue is 1 to 10, 1 to 5, or 1 to 3, for example, 1 or 2;
(9'-b) a hNAGLU mutant in which an amino acid residue constituting the amino acid sequence is deleted and the number of the deleted amino acid residue is 1 to 10, 1 to 5, or 1 to 3, for example, 1 or 2;
(9'-c) a hNAGLU mutant having both of the replacement in the above 9'-a and the deletion in the above 9'-b;
(9'-d) a hNAGLU mutant in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
(9'-e) a hNAGLU mutant having both of the replacement in the above 9'-a and the addition in the above 9'-d;
(9'-f) a hNAGLU mutant having both of the deletion in the above 9'-b and the addition in the above 9'-d;
(9'-g) a hNAGLU mutant having all of the replacement in the above 9'-a, the deletion in the above 9'-b, and the addition in the above 9'-d; and
(9'-h) a hNAGLU mutant showing 80% or more, 85% or more, 90% or more, or 95% or more identity, or 98% or more or 99% identity to the amino acid sequence.

12. A hNAGLU mutant containing a mutation relative to the hNAGLU mutant containing the amino acid sequence represented by SEQ ID NO: 29 according to claim 9 while arginine at position 209, proline at position 320, and aspartic acid at position 321 of an amino acid sequence retained, selected from the group consisting of (10'-a) to (10'-h) below:
(10'-a) a hNAGLU mutant in which an amino acid residue constituting the amino acid sequence is replaced with another amino acid residue and the number of the replaced amino acid residue is 1 to 10, 1 to 5, or 1 to 3, for example, 1 or 2;
(10'-b) a hNAGLU mutant in which an amino acid residue constituting the amino acid sequence is deleted and the number of the deleted amino acid residue is 1 to 10, 1 to 5, or 1 to 3, for example, 1 or 2;
(10'-c) a hNAGLU mutant having both of the replacement in the above 10'-a and the deletion in the above 10'-b;
(10'-d) a hNAGLU mutant in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
(10'-e) a hNAGLU mutant having both of the replacement in the above 10'-a and the addition in the above 10'-d;
(10'-f) a hNAGLU mutant having both of the deletion in the above 10'-b and the addition in the above 10'-d;
(10'-g) a hNAGLU mutant having all of the replacement in the above 10'-a, the deletion in the above 10'-b, and the addition in the above 10'-d; and
(10'-h) a hNAGLU mutant showing 80% or more, 85% or more, 90% or more, or 95% or more identity, or 98% or more or 99% identity to the amino acid sequence.

13. A hNAGLU mutant containing a mutation relative to the hNAGLU mutant containing the amino acid sequence represented by SEQ ID NO: 31 according to claim 9 while arginine at position 210, glutamic acid at position 36, serine at position 37, and serine at position 45 of the amino acid sequence retained, selected from the group consisting of (11'-a) to (11'-h) below:
(11'-a) a hNAGLU mutant in which an amino acid residue constituting the amino acid sequence is replaced with another amino acid residue and the number of the replaced amino acid residue is 1 to 10, 1 to 5, or 1 to 3, for example, 1 or 2;
(11'-b) a hNAGLU mutant in which an amino acid residue constituting the amino acid sequence is deleted and the number of the deleted amino acid residue is 1 to 10, 1 to 5, or 1 to 3, for example, 1 or 2;
(11'-c) a hNAGLU mutant having both of the replacement in the above 11'-a and the deletion in the above 11'-b;
(11'-d) a hNAGLU mutant in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
(11'-e) a hNAGLU mutant having both of the replacement in the above 11'-a and the addition in the above 11'-d;
(11'-f) a hNAGLU mutant having both of the deletion in the above 11'-b and the addition in the above 11'-d;
(11'-g) a hNAGLU mutant having all of the replacement in the above 11'-a, the deletion in the above 11'-b, and the addition in the above 11'-d; and
(11'-h) a hNAGLU mutant showing 80% or more, 85% or more, 90% or more, or 95% or more identity, or 98% or more or 99% identity to the amino acid sequence.

14. A hNAGLU mutant containing a mutation relative to the hNAGLU mutant containing the amino acid sequence represented by SEQ ID NO: 33 according to claim 9 while arginine at position 209, isoleucine at position 54, and lysine at position 620 of the amino acid sequence retained, selected from the group consisting of (12'-a) to (12'-h) below:
(12'-a) a hNAGLU mutant in which an amino acid residue constituting the amino acid sequence is replaced with another amino acid residue and the number of the replaced amino acid residue is 1 to 10, 1 to 5, or 1 to 3, for example, 1 or 2;
(12'-b) a hNAGLU mutant in which an amino acid residue constituting the amino acid sequence is deleted and the number of the deleted amino acid residue is 1 to 10, 1 to 5, or 1 to 3, for example, 1 or 2;
(12'-c) a hNAGLU mutant having both of the replacement in the above 12'-a and the deletion in the above 12'-b;
(12'-d) a hNAGLU mutant in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
(12'-e) a hNAGLU mutant having both of the replacement in the above 12'-a and the addition in the above 12'-d;
(12'-f) a hNAGLU mutant having both of the deletion in the above 12'-b and the addition in the above 12'-d;
(12'-g) a hNAGLU mutant having all of the replacement in the above 12'-a, the deletion in the above 12'-b, and the addition in the above 12'-d; and
(12'-h) a hNAGLU mutant showing 80% or more, 85% or more, 90% or more, or 95% or more identity, or 98% or more or 99% identity to the amino acid sequence.

15. A hNAGLU mutant containing a mutation relative to the hNAGLU mutant containing the amino acid sequence represented by SEQ ID NO: 35 according to claim 9 while arginine at position 210, isoleucine at position 55, and serine at position 45 of the amino acid sequence retained, selected from the group consisting of (13'-a) to (13'-h) below:
(13'-a) a hNAGLU mutant in which an amino acid residue constituting the amino acid sequence is replaced with another amino acid residue and the number of the replaced amino acid residue is 1 to 10, 1 to 5, or 1 to 3, for example, 1 or 2;
(13'-b) a hNAGLU mutant in which an amino acid residue constituting the amino acid sequence is deleted and the number of the deleted amino acid residue is 1 to 10, 1 to 5, or 1 to 3, for example, 1 or 2;
(13'-c) a hNAGLU mutant having both of the replacement in the above 13'-a and the deletion in the above 13'-b;
(13'-d) a hNAGLU mutant in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
(13'-e) a hNAGLU mutant having both of the replacement in the above 13'-a and the addition in the above 13'-d;
(13'-f) a hNAGLU mutant having both of the deletion in the above 13'-b and the addition in the above 13'-d;
(13'-g) a hNAGLU mutant having all of the replacement in the above 13'-a, the deletion in the above 13'-b, and the addition in the above 13'-d; and
(13'-h) a hNAGLU mutant showing 80% or more, 85% or more, 90% or more, or 95% or more identity, or 98% or more or 99% identity to the amino acid sequence.

16. A hNAGLU mutant containing a mutation relative to the hNAGLU mutant containing the amino acid sequence represented by SEQ ID NO: 37 according to claim 9 while arginine at position 210, lysine at position 621, and serine at position 45 of the amino acid sequence retained, selected from the group consisting of (14'-a) to (14'-h) below:
(14'-a) a hNAGLU mutant in which an amino acid residue constituting the amino acid sequence is replaced with another amino acid residue and the number of the replaced amino acid residue is 1 to 10, 1 to 5, or 1 to 3, for example, 1 or 2;
(14'-b) a hNAGLU mutant in which an amino acid residue constituting the amino acid sequence is deleted and the number of the deleted amino acid residue is 1 to 10, 1 to 5, or 1 to 3, for example, 1 or 2;
(14'-c) a hNAGLU mutant having both of the replacement in the above 14'-a and the deletion in the above 14'-b;
(14'-d) a hNAGLU mutant in which one or more amino acid residues are added into the amino acid sequence or to an N-terminus side or a C-terminus side of the amino acid sequence and the number of amino acid residues added is 1 to 10, 1 to 5, or 1 to 3, for example, 1 or 2;
(14'-e) a hNAGLU mutant having both of the replacement in the above 14'-a and the addition in the above 14'-d;
(14'-f) a hNAGLU mutant having both of the deletion in the above 14'-b and the addition in the above 14'-d;
(14'-g) a hNAGLU mutant having all of the replacement in the above 14'-a, the deletion in the above 14'-b, and the addition in the above 14'-d; and
(14'-h) a hNAGLU mutant showing 80% or more, 85% or more, 90% or more, or 95% or more identity, or 98% or more or 99% identity to the amino acid sequence.

17. A hNAGLU mutant containing a mutation relative to the hNAGLU mutant containing the amino acid sequence represented by SEQ ID NO: 39 according to claim 9 while arginine at position 210, isoleucine at position 55, lysine at position 621, and serine at position 45 of the amino acid sequence retained, selected from the group consisting of (15'-a) to (15'-h) below:
(15'-a) a hNAGLU mutant in which an amino acid residue constituting the amino acid sequence is replaced with another amino acid residue and the number of the replaced amino acid residue is 1 to 10, 1 to 5, or 1 to 3, for example, 1 or 2;
(15'-b) a hNAGLU mutant in which an amino acid residue constituting the amino acid sequence is deleted and the number of the deleted amino acid residue is 1 to 10, 1 to 5, or 1 to 3, for example, 1 or 2;
(15'-c) a hNAGLU mutant having both of the replacement in the above 15'-a and the deletion in the above 15'-b;
(15'-d) a hNAGLU mutant in which one or more amino acid residues are added into the amino acid sequence or to an N-terminus side or a C-terminus side of the amino acid sequence and the number of the amino acid residue added is 1 to 10, 1 to 5, or 1 to 3, for example, 1 or 2;
(15'-e) a hNAGLU mutant having both of the replacement in the above 15'-a and the addition in the above 15'-d;
(15'-f) a hNAGLU mutant having both of the deletion in the above 15'-b and the addition in the above 15'-d;
(15'-g) a hNAGLU mutant having all of the replacement in the above 15'-a, the deletion in the above 15'-b, and the addition in the above 15'-d; and
(15'-h) a hNAGLU mutant showing 80% or more, 85% or more, 90% or more, or 95% or more identity, or 98% or more or 99% identity to the amino acid sequence.

18. A DNA comprising:
a gene encoding the hNAGLU mutant according to any one of claims 1 to 17.

19. An expression vector comprising:
the DNA according to claim 18.

20. A mammalian cell transformed with the expression vector according to claim 19.

21. A method for producing a hNAGLU mutant, comprising:
a step of culturing the mammalian cell according to claim 20 in a serum-free medium.

22. A fusion protein of the hNAGLU mutant according to any one of claims 1 to 17 and an antibody, wherein
the antibody binds to a receptor on cerebrovascular endothelial cells such that the fusion protein is capable of crossing a blood-brain barrier (BBB).

23. The fusion protein according to claim 22, wherein
the receptor on cerebrovascular endothelial cells is selected from the group consisting of an insulin receptor, a transferrin receptor, a leptin receptor, a lipoprotein receptor, and an IGF receptor.

24. The fusion protein according to claim 22, wherein
the receptor on cerebrovascular endothelial cells is the transferrin receptor.

25. The fusion protein according to any one of claims 22 to 24, wherein
the antibody is a Fab antibody, a F(ab')₂ antibody, a F(ab') antibody, a single domain antibody, a single chain antibody, or an Fc antibody.

26. The fusion protein according to any one of claims 22 to 25, wherein
the hNAGLU mutant binds to either the C-terminus side or the N-terminus side of a light chain of the antibody.

27. The fusion protein according to any one of claims 22 to 25, wherein
the hNAGLU mutant binds to either the C-terminus side or the N-terminus side of a heavy chain of the antibody.

28. The fusion protein according to any one of claims 22 to 27, wherein
the hNAGLU mutant binds to either the C-terminus side or the N-terminus side of the light chain, or either the C-terminus side or the N-terminus side of the heavy chain of the antibody, via a linker sequence.

29. The fusion protein according to claim 28, wherein
the linker sequence consists of 1 to 50 amino acid residues.

30. The fusion protein according to claim 29, wherein
the linker sequence includes an amino acid sequence selected from the group consisting of one glycine, one serine, an amino acid sequence Gly-Ser, an amino acid sequence Ser-Ser, an amino acid sequence Gly-Gly-Ser, an amino acid sequence represented by SEQ ID NO: 3, an amino acid sequence represented by SEQ ID NO: 4, an amino acid sequence represented by SEQ ID NO: 5, and an amino acid sequence consisting of 1 to 10 of consecutively-linked aforementioned amino acid sequences.

31. A DNA comprising:
a gene encoding the fusion protein according to any one of claims 22 to 30.

32. An expression vector comprising:
the DNA according to claim 31.

33. A mammalian cell transformed with the expression vector according to claim 32.

34. A method for producing a fusion protein of a hNAGLU mutant and an antibody, comprising:
a step of culturing the mammalian cell according to claim 33 in a serum-free medium.
